# EUROPEAN PATENT SPECIFICATION

(11) **EP 0 202 359 B1**
(45) Date of publication and mention of the grant of the patent: **12.01.1994**
(21) Application number: 85108192.7
(22) Date of filing: 02.07.1985
(51) Int. Cl.: A61K 7/20, B65D 35/00

(54) **Dental preparation, article and method for storage and delivery thereof**
Zahnpflegemittel, Vorrichtung und Verfahren zur Lagerung und Verabreichung desselben
Composition dentaire, article et méthode de stockage et d'administration de celle-ci

(30) Priority: 23.05.1985 US 737157; 17.06.1985 US 745993
(43) Date of publication of application: 26.11.1986
(73) Proprietor: BLOCK DRUG COMPANY INC., Jersey City New Jersey (US)
(72) Inventor: Schaeffer, Hans A., Linden, New Jersey (US)
(74) Representative: Grünecker, Kinkeldey, Stockmair & Schwanhäusser Anwaltssozietät

(56) References cited:
- FR-A- 944 506
- US-A- 4 528 180

## Description

This invention relates to a dental preparation useful in the treatment of gum disease, to a method of storing and delivering such preparation to a use point and to an article for the storage and delivery of such preparation.

It has long been recognized that the combination of hydrogen peroxide solution with sodium bicarbonate and table salt has an excellent curative and preventive effect on gum disease caused by bacterial infection. FR-A-944 506 relates to a two part dentifrice composition including a carbonate or bicarbonate component and a peroxide component, in particular to a paste and liquid dentifrice containing these two different compositions in stoichiometric equilibrium, which are kept in two separate containers and when contacted with each other react to produce on the teeth during brushing useful effects, such as the production of active components, detergent-active foams, the prevention of coloration and color change of teeth as well as of the elimination and bleaching of teeth.

Dr. Paul H. Keyes has advocated use of the above combination to the dental profession and to the public at large based on his work of more than 25 years on the subject, which has shown that upon daily and diligent topical application of these materials, gum disease may be effectively controlled. On the basis of his recommendations, many dentists urge their patients to use the Keyes procedure (substantially as described e.g. in S. Elder: "An Alternative To Gum Surgery" Modern Maturity, Aug.-Sept. 1980 pp. 31-32).

Dr. Keyes advocates that a quantity of solid sodium bicarbonate be placed in one hand, and that the toothbrush, held in the other hand, be dipped into a hydrogen peroxide - table salt solution and then transferred to the bicarbonate and applied to the teeth and gums. Upon contact with the gums, the hydrogen peroxide is exposed to the enzyme catalase, which is always present in the buccal cavity, and is attacked thereby resulting in the release of active oxygen. The combination of the active oxygen and the sodium bicarbonate together with table salt destroys the bacteria responsible for gum desease. Unfortunately, hydrogen peroxide and sodium bicarbonate may not be premixed, as they immediately react and are thereby rendered ineffective against gum desease. In addition, hydrogen peroxide is unstable and therefore difficult to store for prolonged periods of time. Finally, mere dipping of the toothbrush in a hydrogen peroxide solution does not insure delivery of a sufficient amount of hydrogen peroxide to the teeth and gums. These factors are responsible for the fact that use of the Keyes procedure is extremely awkward, inconvenient and messy. Another disadvantage stems from the fact that, the mixture of hydrogen peroxide and sodium bicarbonate has a very unpleasant taste. For these reasons, patients have shown extreme reluctance to follow this procedure, especially on a daily basis, as would be required for effective gum disease control. As a result, the benefits which the Keyes procedure affords have largely been left unrealized.

Accordingly, it is the object of this invention to eliminate the above disadvantages associated with use of the Keyes procedure by providing a dental preparation incorporating the active constituents of the Keyes procedure that permits a sufficient, consistent and reproducible amount of hydrogen peroxide to be delivered to the use point, has pleasant taste and is neat and convenient to use, a method for using such preparation that permits contact between hydrogen peroxide and sodium bicarbonate only shortly before use and, therefore, assures maximum effectiveness against gum disease, and an article for the storage and delivery of this improved dental preparation which makes its use neat and convenient and which prevents contact between hydrogen peroxide and sodium bicarbonate prior to application.

In accordance with the present invention, hydrogen or urea peroxide is dissolved in a nontoxic gel for use in combination with a separately stored but substantially simultaneously dispensed paste containing sodium bicarbonate, table (or another suitable) salt, and, preferably, additional cleansing, anticaries and polishing agents as well as an effective concentration of flavoring substances. Each of the gel and paste are loaded either into separate collapsible containers which are connected by means of a common orifice (as in Figure 1), or which have substantially adjacent orifices (as in Fig. 2), or in separate compartments of a single container (as in Figs. 3 or 4). Alternatively, the gel and paste may be loaded in separate compartments of a two-compartment pressurized container (as in Fig. 5) or a mechanically actuated pump, as in Figure 6.

Upon substantially simultaneous squeezing of the containers, in much the same way as common toothpaste tubes (or upon actuating of the pressurized container or pump), controlled quantities of the gel and paste can be simultaneously released onto the toothbrush and immediately applied to the teeth and gums. Control of the peroxide, salt, and NaHCO₃ quantities delivered may be thus effected by specification of the opening of the orifice and the active ingredient concentration in each tube (or pump compartment). As described above, when the brush is applied to teeth and gums, immediate mixing of the products takes place followed by the rapid evolution of active oxygen and carbon dioxide. At the same time, the effervescence accompanying release of active oxygen activates the flavor contained in the bicarbonate paste and produces a lasting highly refreshing taste in the mouth which is unlike any other flavor provided by existing toothpastes or gels.

Another advantage afforded by the present invention, as compared with the Keyes procedure, is that a greater and more consistent amount of peroxide is delivered to the use point.

Yet another advantage stems from the tendency of the present composition to cling to the gum tissues and thus provide them with the full benefit of substantially all of the composition applied to the gums.

Gelling agents suitable for use in preparation of the peroxide gel in accordance with this invention should be nontoxic and neutral to the peroxide to assure its stability. In addition, they should be preferably sensitive to external electrolytes, such as those contained in the sodium bicarbonate paste, in order to make peroxide immediately available to the oral tissues. A gelling agent suitable for use with the present invention is a copolymer of acrylic acid cross-linked with polyallyl sucrose, as described in U.S. Patent No. 2,798,053 issued on July 2, 1957 and assigned to B. F. Goodrich Inc. Other gelling agents resulting in stable hydrogen (or urea) peroxide gels suitable for use in the present invention include those described in British Patent No. 827,331, i.e., organic polymeric acid colloids including polyuronic acids, carboxypolymethylene compounds and polyester resins containing three carboxyl groups, such as partially hydrolized polyacrylates or polymethacrylates and copolymers thereof; and those described in U.S. Patent No. 3,639,574 issued on February 1, 1972 to Schmolka, i.e., polyoxyethylene polyoxypropylene block copolymers, which, according to Schmolka, may be used in the preparation of stable, firm hydrogen peroxide gels. Preferred are water-dispersible copolymers of acrylic acid cross-linked with about 0.75 to about 1.5 % of polyallyl sucrose and neutralized with triethanolamine, NaOH or another alkalizing agent, as taught in U.S. Patent No. 3,499,844¹ issued on March 10, 1970 to Kibbel et al. For purposes of the present invention, Kibbel's acrylic copolymer may be preferably combined with an anionic or non-ionic surfactant, such as disclosed in U.S. Patent No. 4,130,501² issued on December 19, 1978 to Lutz et al. Such surfactants are not essential for the formation of a stable hydrogen peroxide gel in accordance with this invention, but may be added to facilitate distribution and rapid penetration of the active oxygen throughout the area to be treated. A particularly preferred gelling agent for the purposes of the present invention is that described by Kibbel, supra. This gelling agent may but does not have to be modified by the addition of a suitable amount of non-ionic cellulose gum such as hydroxyethyl- or hydroxypropyl-cellulose or hydroxypropyl-methyl-cellulose in order to improve the physical stability of the gel, especially when subjecting it to stress such as that resulting from squeezing of the collapsible tubes, or pumping action.
1 The disclosures of these patents are incorporated herein by reference.
2 The disclosures of these patents are incorporated herein by reference.

The most preferred gelling agents are marketed under the trademark CARBOPOL 941 or 1342 by B.F.Goodrich Company Carbopol 941 does not need neutralization for gelling (and preferably is not neutralized in this invention) because it gels readily in the presence of hydrogen donors. Carbopol 941® has proved to have greater long term physical stability (also believed to be due to hydrogen bonding). Although Carbopol 1342® has just become available on the market and its composition and characteristics have not been fully disclosed, it is claimed by the manufacturer that this acrylic acid copolymer (even though it needs to be neutralized) displays satisfactory long term stability comparable to that of Carbopol 941®.

Gels made from these agents do not need any cellulose additive as a stabilizer, because they are thixotropic (and also pseudoplastic).

Not only is Carbopol 941® the most preferred gelling agent for non-neutralized gels, it is also most preferred for neutralized gels along with Carbopol 934®, 940 and 1342.

The hydrogen peroxide gel may then contain the following ingredients in the following amounts - H₂O₂: 0.1 - 10.0% and preferably 3.0 - 6.5%; Acrylic acid copolymer: 0.05 - 5.0% and, preferably, 1.0 - 3.0%; nonionic cellulose gum (optional): 0 - 2.0% and, preferably, 0.3 - 1.5%; neutralizing agent (triethanolamine, diisopropanolamine, NaOH, KOH): an amount sufficient to raise the gel pH to 3.0 - 6.0; NaOH is preferred. The balance is purified (distilled or deionized) water.

If a non-neutralized gelling agent is used, the aforementioned gel may contain 2 - 80% and preferably 20 - 60% by weight of a polyol selected from the group consisting of glycerin, sorbitol (70% solution), polypropyl glycol, propylene glycol, polyethylene glycol, ethoxylated or propoxylated lower (C₂ - C₅) fatty alcohols and mixtures thereof. The preferred polyol is glycerin. The amount of the water is decreased so that the total adds up to 100% by weight. The pH need not be controlled but falls between 2 and 4.

The sodium bicarbonate paste contains sodium bicarbonate, sodium chloride (or another suitable salt although the salt may be omitted, if desired), purified (distilled or deionized) water, a thickener/stabilizer such as cellulose gum and or magnesium-aluminum silicate, as essential ingredients and, most preferably, it also contains a polishing/stabilizing agent, such as bentonite, silica, titanium dioxide, magnesium oxide or mixtures thereof (the first three and their mixtures are preferred). In order to disperse the "chalky" taste imparted mostly by the bicarbonate and enhance the taste and plasticity of the paste, a bodying agent is added, such as sorbitol, glycerin and/or a glycol. In addition, if the paste (in combination with the gel) is to displace toothpaste completely, additional cleansing agents, such as calcium sulfate, calcium phosphate, hydrated aluminum oxide, calcium carbonate, magnesium carbonate, and magnesium silicate or mixtures thereof can be added. A fluorine-containing compound is also preferably included for its anti-caries activity. Suitable fluorine-containing compounds are NaF, Na-monofluorophosphate, KF, potassium monofluorophosphate, sodium fluorosilicate, sodium fluorozirconate, etc. (with NaF being most preferred). Finally, a foaming agent such as sodium lauryl sulfate (most preferred), sodium N-lauroyl sarcosinate, sodium coconut monoglyceride sulfonate, sodium N-methyl-N-palmitoyl lauride or a nonionic surfactant such as a polysorbate (e.g., Tween 60® or 80® manufactured by ICI Americas, Wilmington, Delaware) or poloxamer or mixtures thereof, which also enhances the peroxide-bicarbonate-salt action, may be added. Flavoring agents, such as sodium saccharin, or other artificial sweeteners, peppermint or spearmint or other flavors are preferably added to further curb the unpleasant taste. Finally, methyl, butyl and/or propyl paraben, sodium benzoate, potassium sorbate or mixtures thereof are preferably added as preservatives, with methyl and propylparaben being most preferred. Use of a coloring agent is optional.

The constituents and quantities for the bicarbonate paste are as follows:
sodium bicarbonate: 2 - 60% and preferably 20 - 40%;
salt: 0 - 6%, preferably 1 - 6% and most preferably 2 - 4% of NaCl (preferred) or KCl, MgCl₂, MgSO₄, Na₂SO₄ or K₂SO₄ or mixtures thereof;
humectant: 2 - 60% and preferably, 15 - 25% consisting of glycerin, sorbitol, propylene glycol, polyethylene glycol, polypropylene glycol, ethoxylated or propoxylated lower fatty alcohols and mixtures thereof;
thickener-stabilizer: nonionic cellulose gum --0.1 - 5% and preferably 1.0 - 2.0%; or magnesium aluminum silicate or mixtures thereof in the same proportions;
stabilizer/polishing agent/cleanser: total 1 - 30%, preferably 1.5 - 20%; these preferably include one or more of: bentonite -- 0.5-7.5%; silica --0.1-8.0%; titanium dioxide -- 0.1-8.0%; and/or magnesium oxide -- 0.2-8.0%; preferably, 1.5-5.0; 0.5-6.0; 0.5-3.0; and 0.5-3.0 percent, respectively.
fluorine-containing compound: sufficient to yield 200 to 3,000 ppm and preferably 1,000 to 2,000 ppm fluorine;
foaming agent: 0.1 - 2.5%; preferably 0.2 - 0.5%;
additional polishing agents: total 1 - 30%, preferably 5 - 20%;
flavoring agent(s): to taste, preferably 0.1 - 2%;
preservatives: 0.05 - 0.5%.

The balance is purified water. A coloring agent may be added. The paste and the gel are preferably used in substantially equal proportions, by volume.

If urea peroxide is used in the gel, the bicarbonate paste composition does not change. The gel composition will be as follows:
urea peroxide: 2 - 25%, preferably 8 - 12%;
acrylic copolymer: 0 - 3.5, preferably 1 - 3%;
glycerin: balance.
The other polyols described above are reactive with the urea peroxide and should not be used.

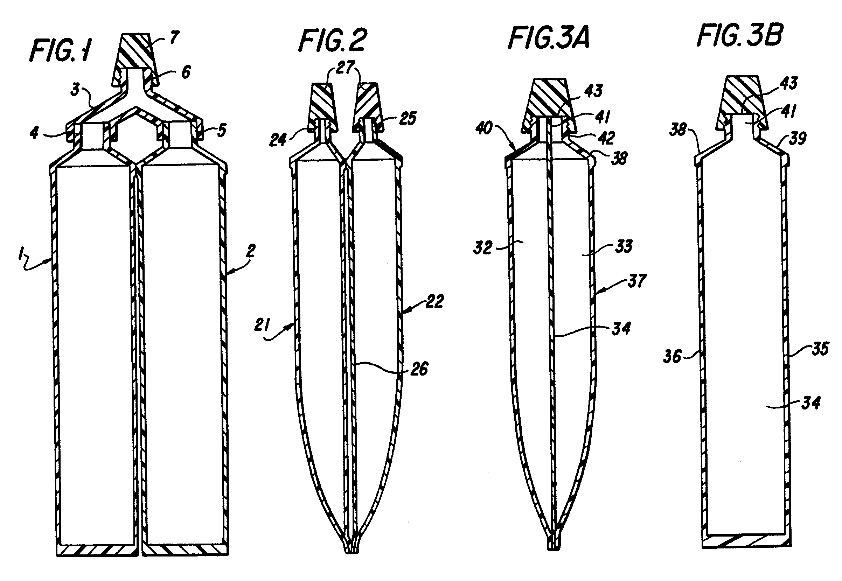

The gel and paste combination may be simultaneously dispensed from separate collapsible tubes preferably made of plastic, or a plastic/metal laminate (to avoid reaction with H₂0₂) and enhanced flavor retention), such as tubes 1 and 2 shown in Figure 1. The tubes are fitted with a Y-shaped conduit 3 which provides them with a common orifice 6. Conduit 3 may also be made of plastic (preferably by injection molding) and is preferably detachably but snugly attached to mouths 4,5 of tubes 1,2 so that it may be removed for cleaning. For additional convenience and in order to ensure dispensation of substantially equal amounts of the gel and paste, the tubes themselves may be held together, e. g., by banding or cementing, along corresponding dorsal sides, shown in Fig. 1, or, preferably, along corresponding ventral sides (see, e. g., Fig. 3A).

Alternatively, the two tubes may be constructed to have a common (preferably flat) sidewall portion 26 as shown in Figure 2. In the latter case, the Y-shaped conduit may be unnecessary, if the mouths 24,25 of tubes 21,22 are sufficiently close so that sufficient quantities of the gel and paste may be simultaneously dispensed directly on the toothbrush. Conventional toothpaste or medicament tubes may be thus used after one of their side walls and the corresponding portion of their head structure are permanently deformed (e.g. by application of pressure) to a substantially flat surface.

A third alternative packaging method involves loading each of the gel and paste into separate compartments of the same collapsible tube, joined by a common orifice, as shown in Figure 3. Composite tube 31 has compartments 32, 33 separated by divider 34 which is firmly attached along substantially diametrically opposed portions 35,36 of the sidewall 37, and corresponding portions 38,39 of head structure 40. Divider 34 may be glued or welded to sidewall 37 and head structure 40 of tube 31 during manufacture of the latter. Divider 34 is preferably provided with protruding portion 41, which extends into the mouth 42 of tube 31 until its edge is substantially flush with rim 43 of mouth 42. Thus, divider 34 forms with sidewall 37 two separate compartments 32,33 of substantially the same volume for storage of the gel and paste, respectively.

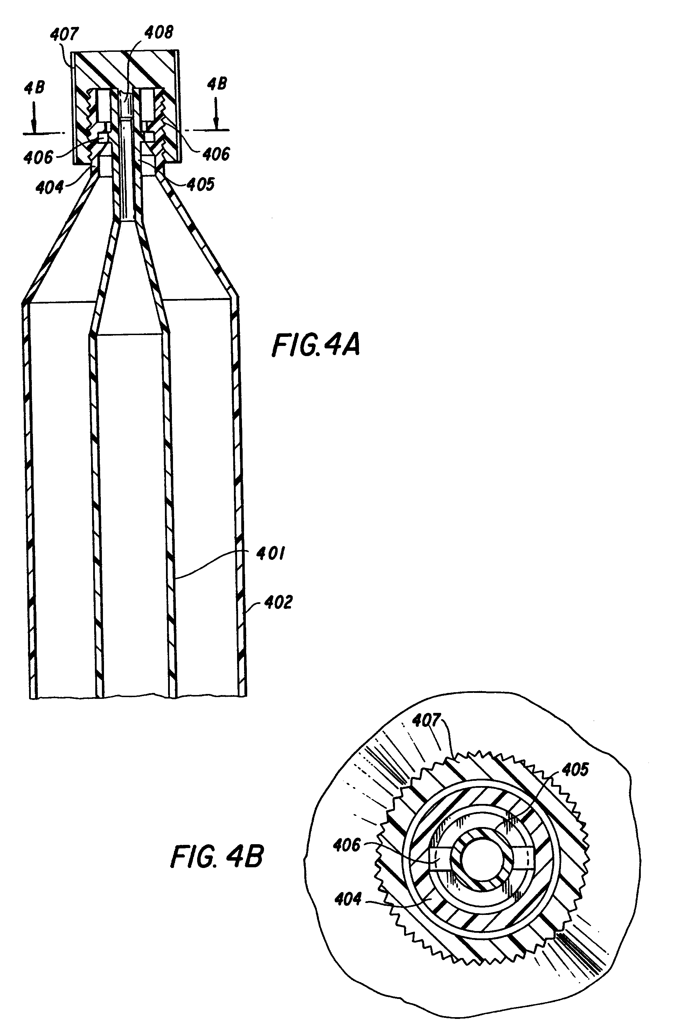

In another alternative packaging method, the two tubes are "concentric" as shown in figure 4A. Inner tube 401 lies within and parallel with outer tube 402.

The mouths of the tubes 401 and 402, designated as 405 and 404 in Figure 4, abut at the same point. Tube 401 is fastened on to tube 402 at the mouth by protrusion 406 (shown in enlargement in Fig. 4B, which is a cross-section of the embodiment of Fig. 4A taken along 4B-4B). Protrusions 406 are inserted in a groove of mouth 404 of tube 402. The material contained in tube 402 can still pass through the available space between mouth 404 of outer tube 402 and mouth 405 of inner tube 401. Engagement of pins 406 in the groove secures the inner tube 401 on the outer tube 402.

The closure 407 of the tube-within-a-tube (which can screw on the outer tube or simply be held by pressure) arrangement may but does not have to be equipped with an interior protrusion 408 to fit in the inner tube in order to prevent premature intermixing of the two components at the mouth of the tube. Because of the pseudoplastic quality of the gel and/or the memory of the plastic tube, however, such intermixing is not likely to occur. The tubes are filled from the bottom and are (subsequently) sealed together by conventional techniques.

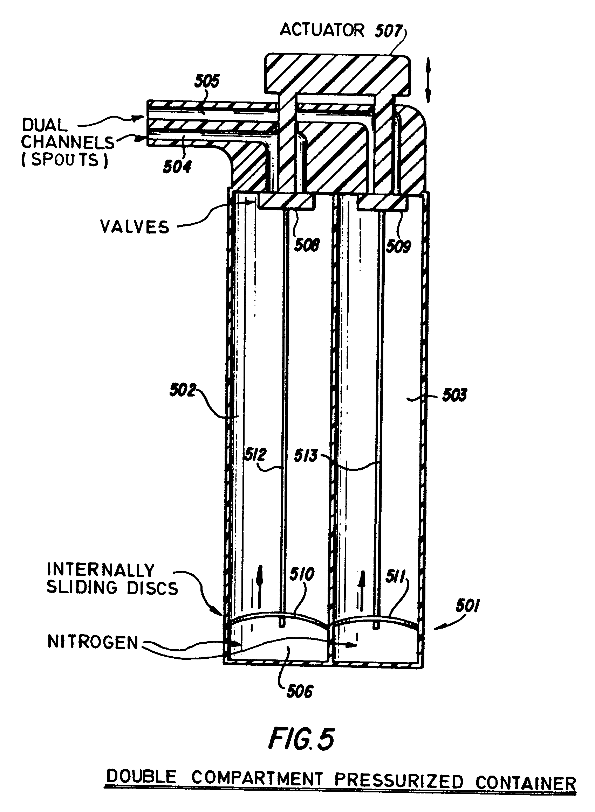

Other alternative packaging arrangements are disclosed in Figures 5 and 6. Pressurized container 501 in Figure 5 is provided with two compartments 502 and 503 and two spouts 504 and 505. The internal pressure of the compartments is maintained by pressurized nitrogen, at the bottom 506 of each compartment. Operation of the mechanical actuator 507 (by pressing downwards) actuates valves 508 and 509 which release the contents of the compartments through the spouts (channels) causing the upwardly slidable sealing disks 510 and 511 (guided by members 512, 513) to move up along the compartments (due to the nitrogen being under pressure). Similar (but conventional) pressurized containers are manufactured for example by American Can company. A dual compartment container, as described above, would be a modification of the existing containers.

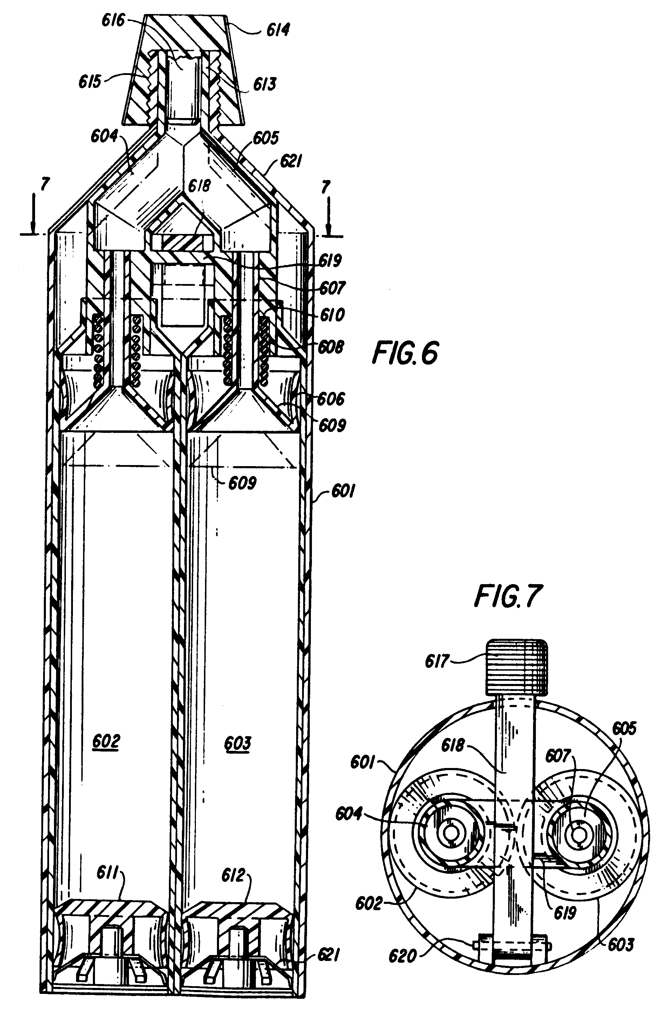

In an alternative pump embodiment depicted in Figs. 6 and 7 a pressurized container 601 is provided with two compartments 602 and 603, and two spouts 604 and 605 for dispensing the gel and paste. Located within the tube 605 is a first piston 606 which is held in place by the upper surface of the contents within compartment 603 and a tubular extension 607 fitting within the lower portion of spout 605. A spring 608 is under compression and is held in position by the upper conical surface 609 of piston 606 and an inner shelf 610 of the spout 605. Lower pistons 611 and 612 are positioned within the lower portions of compartments 602 and 603 respectively so as to follow the dental material upwardly as it is being dispensed into the spouts 604 and 605 and eventually into nozzle 613. The upper part of container 601 has a reduced diameter to encircle the nozzle 613 and provide for a sliding engagement. Outer cap member 614 is threadedly engaged as at 615, with outer surface of nozzle 613 to effectively seal the container and prevent inadvertent dispensing of dental material as well as a pin 616 which fits snugly into open end of nozzle 613.

In operation, and with cap 614 removed, the user will depress a push button lever 617 (seen in Fig. 7) located outside the container 601. Lever 617 has substantially flat elongated member 618 which projects between spouts 604 and 605 and presses against wall 619 which bridges both spouts. Lever 617 is pivoted about pivot pin 620 affixed to inner wall of container 601. As lever 617 is depressed, member 618 will force spouts 604 and 605 downwardly and subsequently spring 608 as well as piston 606 will be lowered to phantom position (as seen in Fig. 6) causing dental material to flow upwardly within extension 607 and spout 605, mixing with material in spout 604 and through nozzle 613 to the bristles of a toothbrush. As the lever is released, the spring 608 will force nozzle 605 upwardly to its original position against conical portion 621 of container 601. The vacuum created will cause piston 606 to raise upwardly and concomitantly cause lower piston 612 to travel upwardly the distance of the expelled dental material. A spring clip 621 slideably engages inner surface of compartment 603 to allow piston to travel upwardly but be prevented from movement downwardly. Description of compartment 602 and spout 604 with its accompanying component parts operate in a like manner as described above.

The particular packaging arrangement used is not important. Those skilled in the art will be able to fashion several obvious modifications of the containers described herein by way of illustration.

Tubes, such as those suitable for use in accordance with the present invention are usually extruded around a cylindrical mandrel, cut into tube segments of suitable length, fitted with head structures and then filled from the bottom and pressed and/or welded closed, substantially as described in, e.g., U.S. Patent No. 4,060,179 issued on November 29, 1977 to McGhie, the disclosure of which is incorporated herein by reference.

In the case of the tube-within-a-tube embodiment of the present invention, the outer tube is provided first but it is not closed at the end opposite to that of the closure. The inner tube (also open-ended at the corresponding end) is inserted and fastened to the mouth of the outer tube. The two tubes are then filled and sealed together. A similar tube-within-a-tube arrangement has been proposed and described in U.S. Patent No. 1,566,218 of Leland and issued on December 15, 1925.

The invention is further illustrated by the following specific examples which are designed merely to illustrate the present invention and not to limit its scope.

In these examples, a hydrogen peroxide gel containing 3 - 6.5% hydrogen peroxide by weight, useful for simultaneous administration with a sodium bicarbonate paste is prepared as follows:

### EXAMPLE 1

### Ingredients

| | |
|---|---|
| Hydrogen peroxide, 35% aqueous solution (5% H₂O₂ in final gel) | 14.3 parts |
| Purified water | 84.45 |
| Copolymer of acrylic acid crosslinked with 1% by weight of polyallyl sucrose having 5.8 allyl groups per molecule (CARBOPOL 934® made by B.F. Goodrich Chemical Co., Akron,Ohio) | 0.5 |
| Hydroxyethyl cellulose | 0.5 |
| Triethanolamine | 0.25 |

The gel is prepared by combining the hydrogen peroxide solution with the purified water, followed by the gradual addition of CARBOPOL 934®. Upon thorough dispersion of the copolymer hydroxyethyl cellulose is slowly added and dissolved. Finally, triethanolamine is added, forming a clear, homogeneous, stable and viscous gel having a pH of 3.4.

### EXAMPLE 2

### Ingredients

| | |
|---|---|
| Hydrogen peroxide, 35% aq. solution (3.5% H₂0₂ in final gel) | 10.0 parts |
| Distilled or deionized water | 88.9 |
| Acrylic acid copolymer CARBOPOL 940® (Goodrich) | 0.6 |
| Hydroxyethylcellulose | 0.5 |
| Sodium hydroxide, 10% solution | q.s. pH 3.8 - 4.0 |

Preparation: same as that of Example 1.

### EXAMPLE 3

### Ingredients

| | |
|---|---|
| Hydrogen peroxide, 35% (3.5% H₂0₂ in final gel) | 10.0 parts |
| Distilled or deionized water | 89.0 |
| Acrylic acid copolymer - CARBOPOL 941® (Goodrich) | 0.7 |
| Hydroxypropylcellulose | 0.3 |
| Sodium hydroxide, 10% solution | q.s. pH 3.8 - 4.0 |

Preparation: same as that of Example 1.

### EXAMPLE 4

### Ingredients

| | |
|---|---|
| Hydrogen peroxide, 35% (4.0% H₂0₂ in final gel) | 11.5 parts |
| Distilled or deionized water | 86.65 |
| Acrylic acid copolymer - CARBOPOL 934® (Goodrich) | 0.75 |
| Sodium laurylsulfate, dentifrice grade | 0.50 |
| Hydroxypropylcellulose | 0.6 |
| Sodium hydroxide, 10% solution | q.s. pH 3.5 - 4.5 |

Preparation:
The hydrogen peroxide solution is combined with the distilled or deionized water. Sodium laurylsulfate is added under constant agitation and dissolved. Gradually, CARBOPOL 934® is added and dispersed. Hydroxypropylcellulose is added in increments and dissolved. When the mixture is homogeneous, sodium hydroxide is added slowly to the desired pH level and viscosity.

### EXAMPLE 5

### Ingredients

| | |
|---|---|
| Hydrogen peroxide, 35% (6.0% H₂0₂ in final gel) | 17.14 parts |
| Distilled or deionized water | 81.76 |
| Acrylic acid copolymer - CARBOPOL 940® (Goodrich) | 0.70 |
| Hydroxyethylcellulose | 0.40 |
| Sodium hydroxide, 10% solution | q.s. pH 3.5-4.0 |

Preparation: same as that of Example 1.

### EXAMPLE 6

### Ingredients

| | |
|---|---|
| Hydrogen peroxide, 35% (3.0% H₂0₂ in final gel) | 8.58 parts |
| Distilled or deionized water | 89.22 |
| Acrylic acid copolymer - CARBOPOL 934® (Goodrich) | 0.70 |
| Hydroxypropyl methylcellulose | 0.65 |
| Nonionic surfactant PLURONIC F 127® (BASF Corp., New Jersey) | 0.85 |
| Sodium hydroxide, 10% solution | q.s. pH 3.5 - 4.5 |

Preparation: same as that of Example 4.

### EXAMPLE 7

The sodium bicarbonate paste is prepared as follows:

### Ingredients

| | |
|---|---|
| Deionized water | 31.94 parts |
| Sorbitol 70% solution, USP | 20.0 |
| Cellulose gum - CMC 7MF® (Hercules) | 1.44 |
| Sodium saccharin | 0.20 |
| Magnesium aluminum silicate - VEEGUM F® (made by R. T. Vanderbilt Co., Inc., Norwalk, Conn.) | 1.17 |
| Sodium bicarbonate, fine powder | 40.00 |
| Sodium chloride | 40.00 |
| Sodium lauryl sulfate - dentifrice grade | 0.30 |
| Peppermint/Spearmint Flavor | 0.75 |
| Methyl paraben, USP | 0.15 |
| Propyl paraben, USP | 0.05 |

Procedure:
Glycerin and propylene glycol are combined in a first container with agitation. Cellulose gum is added and dispersed thoroughly throughout the mixture. Saccharin, methylparaben and propylparaben are added to the required amount of purified water in a separate container and heated to dissolve. VEEGUM F® is added and the mixture is agitated until uniform. The contents of the first container are slowly added to the second container and the final mixture is agitated thoroughly until uniform. Flavoring agent, sodium lauryl sulfate and coloring (if desired) are added and the paste is agitated at moderate speed until uniform. Entrapped air is removed by degassing in a vacuum vessel. Further homogeneity may be obtained by milling, if necessary.

### EXAMPLE 8

### Ingredients

| | |
|---|---|
| Deionized water | 33.43 parts |
| Glycerin | 10.00 |

Glycerin and propylene glycol are combined in a first container with agitation. Cellulose gum is added and dispersed thoroughly throughout the mixture. Saccharin, methylparaben and propylparaben are added in a separate container and heated to dissolve. VEEGUM F® is added and the mixture is agitated until uniform. The contents of the first container are slowly added to the second container and the final mixture is agitated thoroughly until uniform. Flavoring agent, sodium lauryl sulfate and coloring (if desired) are added and the paste is agitated at moderate speed until uniform. Entrapped air is removed by degassing in a vacuum vessel. Further homogeneity may be obtained by milling, if necessary.

### EXAMPLE 8

### Ingredients

| | |
|---|---|
| Deionized water | 33.43 parts |
| Glycerin | 10.00 |
| Propylene glycol | 10.0 |
| Cellulose gum - CMC 7MF® (Hercules) | 1.45 |
| Sodium saccharin | 0.20 |
| Magnesium aluminum silicate - VEEGUM F® | 1.17 |
| Sodium bicarbonate, fine powder | 25.00 |
| Dicalcium phosphate dihydrate | 13.50 |
| Dicalcium phosphate, anhydrous | 1.50 |
| Sodium chloride | 2.50 |
| Sodium lauryl sulfate, dentifrice grade | 0.30 |
| Methylparaben, USP | 0.15 |
| Propylparaben, USP | 0.05 |
| Peppermint/Spearmint Flavor | 0.75 |
| FD & C Blue No. 1, 0.1% solution | q.s. |
| DS & C Yellow No. 6, 0.1% solution | q.s. |

Procedure: Same as that of Example 8.

### EXAMPLE 9

Paste containing fluoride:

### Ingredients

| | |
|---|---|
| Deionized water | 33.51 |
| Sorbitol, 70% solution | 20.00 |
| Sodium saccharin | 0.20 |
| Cellulose gum CMC 7MF® (Hercules) | 1.54 |
| Magnesium aluminum silicate - VEEGUM F® | 1.17 |
| Sodium fluoride | 0.33 |
| Methyl paraben, USP | 0.15 |
| Propyl paraben, USP | 0.05 |
| Calcium sulfate | 10.00 |
| Sodium bicarbonate | 25.00 |
| Sodium chloride | 2.00 |
| Hydrated aluminum oxide | 5.00 |
| Peppermint/Spearmint Flavor | 0.75 |
| Sodium lauryl sulfate | 0.30 |

Procedure: Same as that of Example 8.

### EXAMPLE 10

### Peroxide Gels

### Composition 10-A

| | |
|---|---|
| Hydrogen peroxide, 35% aquous solution (4.0% H₂O₂ in final gel) | 11.5 |
| Distilled water | 86.6 |
| Acrylic acid copolymer - Carbopol 934® (Goodrich) | 1.5 |
| Sodium lauryl sulfate, dentifrice grade | 0.1 |
| Hydroxypropyl cellulose | 0.3 |
| Sodium hydroxide, 10% solution | q.s. pH 3.0 - 4.5 |

### Composition 10-B

| | |
|---|---|
| Hydrogen peroxide, 35% (4.0% H₂O₂ in final gel) | 11.5 |
| Distilled water | 88.0 |
| Acrylic acid copolymer (Carbopol 934®, 940®, 941®, or 1342®) | 1.5 |
| Sodium hydroxide, 10% solution | q.s. pH 3.0 - 4.5 |

### Composition 10-C

| | |
|---|---|
| Hydrogen peroxide, 35% (4.0% H₂O₂ in final gel) | 11.5 |
| Distilled water | 46.0 |
| Glycerin, anhydrous | 40.0 |
| Acrylic acid copolymer - Carbopol 941® | 2.5 |

### Methods of preparation

Composition 10-A: same as listed in Example 4
Composition 10-B: same as in Example 4 except that sod. lauryl sulfate and hydroxypropyl cellulose were omitted. The composition including Carbopol 1342® has not been actually made.
Composition 10-C: The glycerin and water were combined and heated to 50 - 60°C. Very slowly, Carbopol 941® was added under constant agitation.

When a clear gel had formed and no undissolved lumps remained, the gel was cooled to 25°C and the hydrogen peroxide was added. Agitation was maintained until the mixture became homogeneous. The gel was de-aerated in a vacuum vessel.

### EXAMPLE 11: Sodium bicarbonate Paste

### Composition 11-A

| | |
|---|---|
| Glycerin | 25.0 |
| Cellulose gum CMC 7MF® (Hercules) | 1.54 |
| Deionized water | 32.71 |
| Magnesium aluminum silicate Veegum F® (R.T. Vanderbilt) | 1.10 |
| Sodium saccharin | 0.60 |
| Sodium chloride | 2.0 |
| Methyl paraben | 0.15 |
| Propyl paraben | 0.05 |
| (Sodium hydroxide solution 10%, may be added, if necessary for pH adjustment) | q.s. pH 8.0 - 8.5, |
| Sodium fluoride | 0.22 |
| Bentonite | 4.0 |
| Titanium dioxide | 2.0 |
| Silica | 4.0 |
| Sodium bicarbonate | 25.0 |
| Flavor (spearmint) | 1.0 |
| Sodium lauryl sulfate | 0.3 |
| Color (FDC Blue No. 1) | q.s. |

### Method of preparation

The cellulose gum was added to the glycerin and dispersed thoroughly.

In a separate vessel, the parabens, sodium saccharin, and sodium chloride were dissolved in water at 60 - 70°C. To the clear solution was added the Veegum F® and the mixture was agitated until uniform. The pH of this solution was determined to be 8.0 - 8.5 (and adjusted, if necessary).

The gum dispersion was added to the Veegum F® solution and agitated until uniform.

To the blend were added the powders, bentonite, TiO₂, silica, NaHCO₃, and NaF, under vigorous agitation.

To the paste were added flavor, sodium lauryl sulfate and color.

The finished paste is milled and degassed in a vacuum vessel.

### EXAMPLE 13

### Urea peroxide gel

### Composition 13-A

| | |
|---|---|
| Urea peroxide (35% H₂O₂ equivalent) | 10.0 parts by weight |
| Glycerin, anhydrous | 90.0 |

Method: Urea peroxide is slowly added under agitation to the anhydrous glycerin until a clear gel is formed which has a suitable consistency for filling into collapsible tubes.

### Composition 13-B

| | |
|---|---|
| Urea peroxide | 10.0 |
| Acrylic acid copolymers - Carbopol 941® | 1.5 |
| Glycerin, anhydrous | 88.5 |

The method is the same as that for 13-A except the urea peroxide and Carbopol 941® are both added to the glycerin solution.

## Claims

1. A composition useful in combating gum disease comprising:
(a) a gel component comprising (i) 0.1 to 10% by weight of hydrogen peroxide; (ii) 0.05 to 5.0% by weight of a water-dispersible copolymer of acrylic acid cross-linked with polyallyl sucrose; (iii) 0.0 to 2.0% by weight of a nonionic cellulose stabilizer; (iv) a neutralizing agent selected from the group consisting of sodium hydroxide, potassium hydroxide, triethanolamine, diisopropylamine and ammonia in an amount sufficient to raise the gel pH to about 3 to 6; and (v) purified water;
(b) a paste component comprising: (i) 2 to 60% sodium bicarbonate; (ii) 0 to 6% of a salt selected from the group consisting of NaCl, KCl, MgCl₂, MgSO₄, Na₂SO₄, and K₂SO₄; (iii) 2 to 60% of a humectant selected from the group consisting of glycerin, sorbitol, polyethylene glycol, proplylene glycol, polypropylene glycol, an ethoxylated lower fatty alcohol, a propoxylated lower fatty alcohol and mixtures thereof; (iv) 0.1 to 5% of a thickener stabilizer selected from the group consisting of cellulose gum, magnesium aluminum silicate and mixtures thereof; (v) 1 to 30% of a stabilizing polishing agent selected from the group consisting of bentonite, titanium dioxide, silica, magnesium oxide and mixtures thereof; and (vi) purified water; said paste component and gel component being combined immediately prior to use.

2. The composition of claim 1, said paste component also comprising a fluorine-containing compound selected from the group consisting of NaF, KF, sodium monofluorophosphate, potassium monofluorophosphate, sodium fluorosilicate, sodium fluorozirconate and mixtures thereof in an amount sufficient to yield 200 to 3,000 ppm of fluorine; said composition also being effective against caries.

3. The composition of claim 2, said paste further comprising 0.1 to 2.5% of a foaming agent selected from the group consisting of sodium lauryl sulfate, sodium lauroyl sarcosinate, sodium coconut monoglyceride sulfonate, sodium N-methyl-N-palmitoyl lauride, a polysorbate, a poloxamer and mixtures thereof.

4. The composition of claim 3, said paste further comprising 1 to 30% of an additional cleansing agent selected from the group consisting of calcium sulfate, calcium phosphate, hydrated aluminum oxide, calcium carbonate, magnesium carbonate, magnesium silicate and mixtures thereof.

5. The composition of claim 4 wherein said paste component also comprises a preservative, a coloring agent and a flavoring agent.

6. The composition of claim 1, wherein said gel component comprises 3.0 to 6.5% hydrogen peroxide; 1 to 3% of said copolymer; and 0.3 to 1.5% of said neutralizing agent and wherein said paste component comprises 20 to 40% of said sodium bicarbonate; 2 to 4% of said salt; 15 to 25% of said humectant; 1.0 to 2.0% of said stabilizer; and 1.5 to 20% of said stabilizing polishing agent.

7. A composition useful in combating gum disease comprising:
(a) a non-neutralized gel component consisting essentially of 0.1 to 10.0% of hydrogen peroxide; 0.05 to 5.0% of acrylic acid copolymer cross-linked with polyallyl sucrose; 2 to 80% of a polyol selected from the group consisting of glycerin, sorbitol (70% solution), propylene glycol, polypropylene glycol, polyethylene glycol, an ethoxylated lower fatty alchol, a propoxylated lower fatty alcohol and mixtures thereof; and purified water;
(b) a paste component comprising: (i) 2 to 60% sodium bicarbonate; (ii) 0 to 6% of a salt selected from the group consisting of NaCl, KCl, MgCl₂, MgSO₄, Na₂SO₄, and K₂SO₄; (iii) 2 to 60% of a humectant selected from the group consisting of glycerin, sorbitol, polyethylene glycol, propylene glycol, polypropylene glycol, an ethoxylated lower fatty alcohol, a propoxylated lower fatty alchol and mixtures thereof; (iv) 0.1 to 5% of a thickener stabilizer selected from the group consisting of cellulose gum, magnesium aluminum silicate and mixtures thereof; (v) 1 to 30% of a stabilizing polishing agent selected from the group consisting of bentonite, titanium dioxide, silica, magnesium oxide and mixtures thereof; and (vi) purified water; said paste component being combined with said gel component immediately prior to use.

8. The composition of claim 7, said paste component also comprising a fluorine-containing compound selected from the group consisting of NaF, KF, sodium monofluorophosphate, potassium monofluorophosphate, sodium fluorosilicate, sodium fluorozirconate and mixtures thereof, in an amount sufficient to yield 200 to 3,000 ppm of fluorine.

9. The composition of claim 8, said paste further comprising 0.1 to 2.5% of a foaming agent selected from the group consisting of sodium lauryl sulfate, sodium lauroyl sarcosinate, sodium coconut monoglyceride sulfonate, sodium N-methyl-N-palmitoyl lauride, a polysorbate, a poloxamer and mixtures thereof.

10. The composition of claim 9, said paste further comprising 1 to 30% of an additional cleansing agent selected from the group consisting of calcium sulfate, calcium phosphate, hydrated aluminum oxide, calcium carbonate, magnesium carbonate, magnesium silicate and mixtures thereof.

11. The composition of claim 10 wherein said paste component also comprises a preservative, a coloring agent and a flavoring agent.

12. The composition of claim 7, wherein said gel component contains 3.0 to 6.5% hydrogen peroxide; and 20 to 60% of said polyol and wherein said paste component comprises 20 to 40% of said sodium bicarbonate; 2 to 4% of said salt; 15 to 25% of said humectant; 1.0 to 2.0% of said stabilizer; and 1.5 to 20% of said stabilizing polishing agent.

13. A composition useful for combating gum disease comprising:
(a) a gel component comprising: (i) 2 to 25% urea peroxide; (ii) 0.0 to 5 0% of an acrylic acid copolymer crosslinked with polyallyl sucrose; the balance being glycerin; and
(b) a paste component comprising: (i) 2 to 60% sodium bicarbonate; (ii) 0 to 6% of a salt selected from the group consisting of NaCl, KCl, MgCl₂, MgSO₄, Na₂SO₄, and K₂SO₄; (iii) 2 to 60% of a humectant selected from the group consisting of glycerin, sorbitol, polyethylene glycol, polypropylene glycol, propylene glycol, an ethoxylated lower fatty alcohol, a propoxylated lower fatty alcohol and mixtures thereof; (iv) 0.1 to 5% of a thickener stabilizer selected from the group consisting of cellulose gum, magnesium aluminum silicate and mixtures thereof; (v) 1 to 30% of a stabilizing polishing agent selected from the group consisting of bentonite, titanium dioxide, silica, magnesium oxide and mixtures thereof; and (vi) purified water, said paste and said gel component being combined immediately prior to use.

14. The composition of claim 13, said paste component also comprising a fluorine-containing compound selected from the group consisting of NaF, KF, sodium monofluorophosphate, potassium monofluorophosphate, sodium fluorosilicate, sodium fluorozirconate and mixtures thereof in an amount sufficient to yield 200 to 3,000 ppm of fluorine.

15. The composition of claim 14, said paste further comprising 0.1 to 2.5% of a foaming agent selected from the group consisting of sodium lauryl sulfate, sodium lauroyl sarcosinate, sodium coconut monoglyceride sulfonate, sodium N-methyl-N-palmitoyl lauride, a polysorbate, a poloxamer and mixtures thereof.

16. The composition of claim 15, said paste further comprising 1 to 30% of an additional cleansing agent selected from the group consisting of calcium sulfate, calcium phosphate, hydrated aluminum oxide, calcium carbonate, magnesium carbonate, magnesium silicate and mixtures thereof.

17. The composition of claim 16 wherein said paste component also comprises a preservative, a coloring agent and a flavoring agent.

18. The composition of claim 13 wherein said gel component contains 8 to 12% of urea peroxide; 1 to 3% of said acrylic acid copolymer; and glycerin and wherein said paste component comprises 20 to 40% of said sodium bicarbonate; 2 to 4% of said salt; 15 to 25% of said humectant; 1.0 to 2.0% of said stabilizer; and 1.5 to 20% of said stabilizing polishing agent.

19. The composition of claim 5, wherein said gel copolymer is selected from the group consisting of carboxyvinyl polymers, said gel stabilizer is hydroxypropyl cellulose and said neutralizer is NaOH; said salt is NaCl, said paste humectant is glycerin, said stabilizing polishing agent is selected from the group consisting of TiO₂, silica, bentonite and mixtures thereof; said fluorinated compound is NaF, said foaming agent is sodium lauryl sulfate, and said preservative is selected from the group consisting of methyl and propyl paraben.

20. The composition of claim 11 wherein said gel copolymer is a carboxyvinyl polymer and polyol is glycerin; said salt is NaCl, said paste humectant is glycerin, said stabilizing polishing agent is selcted from the group consisting of TiO₂, silica, bentonite and mixtures thereof; said fluorinated compound is NaF, said foaming agent is sodium lauryl sulfate, and said preservative is selected from the group consisting of methyl and propyl paraben.

21. The composition of claim 17 wherein said polymer is a carboxyvinyl polymer, said salt is NaCl, said paste humectant is glycerin, said stabilizing polishing agent is selected from the group consisting of TiO₂, silica, bentonite and mixtures thereof; said flouroinated compound is NaF, said foaming agent is sodium lauryl sulfate, and said preservative is selected from the group consisting of methyl and propyl paraben.

22. A combination of a two-compartment container and a composition consisting of a gel component and a paste component and contained in said container, said combination being suitable for use in combating gum disease, said container comprising:
(a) a first compartment containing said gel, said gel comprising (i) 1 to 10% of hydrogen peroxide; (ii) 0.05 to 5.0% of a water dispersible copolymer of acrylic acid crosslinked with polyallyl sucrose; (iii) 0.0 to 2.0% of a nonionic cellulose stabilizer; (iv) an amount of a neutralizing agent sufficient to adjust the gel pH to 3.0 to 6.0, said neutralizing agent being selected from the group consisting of NaOH, KOH, triethanolamine, diisopropylamine and ammonia; and (v) water; such that the gel liquifies immediately upon contact with a mildly alkaline environment containing a strong electrolyte, thereby causing the release of bactericidally effective amounts of nascent oxygen; said gel having sufficient viscosity to support itself on the bristles of a toothbrush and sufficient fluidity to be dispensed from said container; said first compartment having an orifice for dispensing controlled amounts of said gel;
(b) a second compartment containing said paste, said paste comprising (i) 2 to 60% by weight of sodium bicarbonate; (ii) 0 to 6% of a salt selected from the group consisting of NaCl, KCl, MgCl₂, MgSO₄, Na₂SO₄, K₂SO₄ and mixtures thereof; (iii) 2 to 60% of a humectant selected from the group consisting of glycerin, sorbitol, polyethylene glycol, polypropylene glycol, an ethoxylated lower fatty alcohol, a propoxylated lower fatty alcohol, and mixtures thereof; (iv) 0.1 to 5.0% of a thickener-stabilizer selected from the group consisting of cellulose gum, magnesium aluminum silicate and mixtures thereof; (v) 1 to 30% of a stabilizing polishing agent selected from the group consisting of bentonite, titanium dioxide, silica, magnesium oxide and mixtures thereof; and (vi) purified water; said paste having sufficient viscosity to support itself on the bristles of a toothbrush and sufficient fluidity to be dispensed from said container, said second compartment having an orifice for dispensing controlled amounts of said paste substantially simultaneously with the dispensation of said gel; said first compartment orifice and said second compartment orifice being adapted to dispense said gel and said paste respectively at the same use point, said first and second compartments having a common wall portion and said orifices being substantially adjacent.

23. A combination of a two compartment container and a composition consisting of a gel component and a paste component, said composition being contained in said container, said combination being suitable for combating gum disease, said container comprising:
(a) a first compartment containing said gel, said gel being a non-neutralized gel and comprising 0.1 to 10% of hydrogen peroxide; 0.05 to 5.0% of an acrylic acid copolymer crosslinked with polyallyl sucrose; 2 to 80% of a polyol selected from the group consisting of glycerin, sorbitol 0 to 70% solution, propylene glycol, polypropylene glycol, polyethylene glycol, an ethoxylated lower fatty alcohol, a propoxylated lower fatty alcohol and mixtures thereof; and purified water; such that the gel liquifies immediately upon contact with a mildly alkaline environment containing a strong electrolyte, thereby causing the release of bactericidally effective amounts of nascent oxygen; said gel having sufficient viscosity to support itself on the bristles of a toothbrush and sufficient fluidity to be dispensed from said container; said first compartment having an orifice for dispensing controlled amounts of said gel;
(b) a second compartment containing said paste, said paste comprising (i) 2 to 60% by weight of sodium bicarbonate; (ii) 0 to 6% of a salt selected from the group consisting of NaCl, KCl, MgCl₂, MgSO₄, Na₂SO₄, K₂SO₄ and mixtures thereof; (iii) 2 to 60% of a humectant selected from the group consisting of glycerin, sorbitol, polyethylene glycol, polypropylene glycol, an ethoxylated lower fatty alcohol, a propoxylated lower fatty alcohol, and mixtures thereof; (iv) 0.1 to 5.0% of a thickener-stabilizer selected from the group consisting of cellulose gum, magnesium aluminum silicate and mixtures thereof; (v) 1 to 30% of a stabilizing polishing agent selected from the group consisting of bentonite, titanium dioxide, silica, magnesium oxide and mixtures thereof; and (vi) purified water; said paste having sufficient viscosity to support itself on the bristles of a toothbrush and sufficient fluidity to be dispensed from said container, said second compartment having an orifice for dispensing controlled amounts of said paste substantially simultaneously with the dispensation of said gel; said first compartment orifice and said second compartment orifice being adapted to dispense said gel and said paste respectively at the same use point, said first and second compartments having a common wall portion and said orifices being substantially adjacent.

24. A combination of a two compartment container and a composition consisting of a gel component and a paste component, said composition being contained in said container, said combination being suitable for combating gum disease, said container comprising:
(a) a first compartment containing said gel, said gel comprising 2 to 25% urea peroxide, 0.0 to 3.5% of an acrylic acid copolymer crosslinked with polyallyl sucrose; and glycerin; such that the gel liquifies immediately upon contact with a mildly alkaline environment containing a strong electrolyte, thereby causing the release of bactericidally effective amounts of nascent oxygen; said gel having sufficient viscosity to support itself on the bristles of a toothbrush and sufficient fluidity to be dispensed from said container; said first compartment having an orifice for dispensing controlled amounts of said gel;
(b) a second compartment containing said paste, said paste comprising (i) 2 to 60% by weight of sodium bicarbonate; (ii) 0 to 6% of a salt selected from the group consisting of NaCl, KCl, MgCl₂, MgSO₄, Na₂SO₄, K₂SO₄ and mixtures thereof; (iii) 2 to 60% of a humectant selected from the group consisting of glycerin, sorbitol, polyethylene glycol, polypropylene glycol, an ethoxylated lower fatty alcohol, a propoxylated lower fatty alcohol, and mixtures thereof; (iv) 0.1 to 5.0% of a thickener-stabilizer selected from the group consisting of cellulose gum, magnesium aluminum silicate and mixtures thereof; (v) 1 to 30% of a stabilizing polishing agent selected from the group consisting of bentonite, titanium dioxide, silica, magnesium oxide and mixtures thereof; and (vi) purified water; said paste having sufficient viscosity to support itself on the bristles of a toothbrush and sufficient fluidity to be dispensed from said container, said second compartment having an orifice for dispensing controlled amounts of said paste substantially simultaneously with the dispensation of said gel; said first compartment orifice and said second compartment orifice being adapted to dispense said gel and said paste respectively at the same use point, said first and second compartments having a common wall portion and said orifice being substantially adjacent.

25. The combination of claim 22 wherein said gel contains greater than 1.2 and up to 5.0% of said copolymer and said two-compartment container is selected from the group consisting of two-compartment collapsible tubes with flexible sidewalls, two-compartment pressurized containers, two-compartment pumps, and combinations of two single-compartment tubes.

26. The combination of claim 23, wherein said two-compartment container is selected from the group consisting of two-compartment collapsible tubes with flexible sidewalls, two-compartment pressurized containers, two-compartment pumps, and combinations of two single-compartment tubes.

27. The combination of claim 24, wherein said two-compartment container is selected from the group consisting of two-compartment collapsible tubes with flexible sidewalls, two-compartment pressurized containers, two-compartment pumps, and combinations of two single-compartment tubes.

28. A composition useful in combating gum disease comprising:
(a) a gel component comprising an effective amount of peroxide selected from the group consisting of hydrogen peroxide and urea peroxide; a water dispersible polymer selected from the group consisting of a copolymer of acrylic acid cross-linked with polyallyl sucrose, a polyuronic acid colloid, a carboxymethylene colloid, a partially hydrolyzed polacrylate, a partially hydrolyzed polymethacrylate, a partially hydrolyzed acrylate-methacrylate copolymer, and a polyoxyethylene-polyoxypropylene block copolymer; a nonionic cellulose gum stabilizer; purified water; and an amount of a neutralizing agent selected from the group consisting of: sodium hydroxide, potassium hydroxide, triethanolamine, diisopropylamine and ammonia sufficient to raise the pH of said gel to within 3 to 6; said gel having sufficient viscosity to support itself on the bristles of a toothbrush and sufficient fluidity to be dispensed from a collapsible sidewall tube upon squeezing; and
(b) a paste component comprising effective amounts of sodium bicarbonate and a salt selected from the group consisting of sodium chloride and magnesium sulfate, at least one thickener-stabilizer selected from the group consisting of cellulose gum and magnesium-aluminum silicate, a bodying agent, purified water, at least one cleansing-polishing agent selected from the group consisting of calcium sulfate, calcium phosphate and hydrated aluminum oxide, and a foaming agent, said paste having sufficient viscosity to support itself on the bristles of a toothbrush and sufficient fluidity to be dispensed from a collapsible sidewall tube by squeezing, said gel and said paste components being mixed immediately prior to use.

29. The composition of claim 28 wherein said gel component comprises 1 to 10% H₂O₂, 0.05 to 1.2% acrylic acid copolymer and 0.1 to 1.5% non-ionic cellulose stabilizer by weight, said cellulose stabilizer being selected from the group consisting of hydroxyethyl cellulose, hydroxypropyl cellulose and hydroxypropyl methylcellulose, and wherein said paste component comprises 10 to 50% sodium bicarbonate, 0 to 6% salt, 1 to 3% of at least one of cellulose gum and magnesium-aluminum silicate, 5 to 30% of a bodying agent, 1 to 40% of said cleanser-polishing agent and 0.1 to 2.5% foaming agent by weight.

30. A method for cleaning teeth comprising extruding a gel component according to claims 1 and 7 comprising hydrogen peroxide as an active ingredient, said first component being suitable for oral use;
extruding a paste component according to claims 1 and 7 comprising sodium bicarbonate as an active ingredient, said second component being suitable for oral use;
placing said first component and said second component in contact with each other on a toothbrush, and
brushing said teeth using said first and second components concurrently as a cleaning medium.

31. The method of claim 30, wherein said first and second components are extruded together on the toothbrush.

32. The method of claim 30, wherein said first and second components are extruded and placed on the brush separately.

33. The method of claim 30, wherein said brushing takes place immediately after extruding said components and placing them onto said toothbrush.

34. The method of claim 30, wherein said first component contains from 0.1 to 10% H₂O₂.

35. The method of claim 30, wherein said first component is a non-neutralized gel.

36. The method of claim 30, wherein said first component is a neutralized gel.

37. The method of claim 30, wherein said second component contains from 2 to 60% sodium bicarbonate by weight.

38. The method of claim 30, wherein said second component is a paste.

39. The method of claim 30, wherein said first component is a gel comprising as a gelling agent a member selected from the group consisting of: (a) copolymers of acrylic acid cross-linked with polyallyl sucrose; (b) an organic polymeric acid colloid; and (c) a polyoxyethylene/polyoxypropylene block copolymer.

40. The method of claim 30, wherein said second component is a paste comprising a thickener/stabilizer.

41. The method of claim 40, wherein said paste component further comprises a bodying agent.

42. The method of claim 40, wherein said paste component further comprises an additional tooth-cleaning agent.

43. The method of claim 40, wherein said paste component further comprises a fluorine-containing compound having anti-caries activity.

44. The method of claim 35, wherein said gel component further comprises a copolymer of acrylic acid cross-linked with polyallyl sucrose as a gelling agent.

45. The method of claim 36, wherein said gel component further comprises a copolymer of acrylic acid cross-linked with polyallyl sucrose as a gelling agent and a polyol selected from the group consisting of glycerin, sorbitol, propylene glycol, polyropylene glycol, polyethylene glycol, an ethoxylated lower fatty alcohol and a propoxylated lower fatty acid alcohol.

## Patentansprüche

1. Zusammensetzung, die bei der Bekämpfung von Zahnfleischerkrankungen brauchbar ist, umfassend:
(a) eine Gelkomponente umfassend (i) 0,1 bis 10 Gew.-% Wasserstoffperoxid; (ii) 0,05 bis 5,0 Gew.-% eines wasserdispergierbaren Copolymers aus mit Polyallylsucrose vernetzter Acrylsäure; (iii) 0,0 bis 2,0 Gew.-% eines nichtionischen Cellulosestabilisators; (iv) ein neutralisierendes Mittel, ausgewählt aus der Gruppe bestehend aus Natriumhydroxid, Kaliumhydroxid, Triethanolamin, Diisopropylamin und Ammoniak in einer Menge, die ausreicht, um den pH-Wert des Gels auf etwa 3 bis 6 anzuheben; und (v) gereinigtes Wasser;
(b) eine Pastenkomponente umfassend: (i) 2 bis 60% Natriumbicarbonat; (ii) 0 bis 6% eines Salzes, ausgewählt aus der Gruppe bestehend aus NaCl, KCl, MgCl₂, MgSO₄, Na₂SO₄ und K₂SO₄; (iii) 2 bis 60% eines Feuchthaltemittels, ausgewählt aus der Gruppe bestehend aus Glycerin, Sorbit, Polyethylenglykol, Propylenglykol, Polypropylenglykol, einem ethoxylierten niederen Fettalkohol, einem propoxylierten niederen Fettalkohol und Gemischen davon; (iv) 0,1 bis 5% eines Verdickungsmittelstabilisators, ausgewählt aus der Gruppe bestehend aus Cellulosegummi, Magnesiumaluminiumsilicat und Gemischen davon; (v) 1 bis 30% eines stabilisierenden Poliermittels ausgewählt aus der Gruppe bestehend aus Bentonit, Titandioxid, Siliciumdioxid, Magnesiumoxid und Gemischen davon; und (vi) gereinigtes Wasser; wobei die Pastenkomponente und Gelkomponente unmittelbar vor der Verwendung vereinigt werden.

2. Zusammensetzung nach Anspruch 1, wobei die Pastenkomponente auch eine fluorhaltige Verbindung, ausgewählt aus der Gruppe bestehend aus NaF, KF, Natriummonofluorphosphat, Kaliummonofluorphosphat, Natriumfluorosilicat, Natriumfluorozirconat und Gemischen davon, in einer Menge umfaßt, die ausreichend ist, um 200 bis 3000 ppm Fluor zu ergeben; wobei die Zusammensetzung auch gegen Karies wirksam ist.

3. Zusammensetzung nach Anspruch 2, wobei die Paste ferner 0,1 bis 2,5% eines Schaummittels, ausgewählt aus der Gruppe bestehend aus Natriumlaurylsulfat, Natriumlauroylsarcosinat, Natrium-Kokosnußmonoglyceridsulfonat, Natrium-N-methyl-N-palmitoyl-laurid, einem Polysorbat, einem Poloxamer und Gemischen davon, umfaßt.

4. Zusammensetzung nach Anspruch 3, worin die Paste ferner 1 bis 30% eines zusätzlichen Reinigungsmittels, ausgewählt aus der Gruppe bestehend aus Calciumsulfat, Calciumphosphat, Aluminiumhydroxid, Calciumcarbonat, Magnesiumcarbonat, Magnesiumsilicat und Gemischen davon, umfaßt.

5. Zusammensetzung nach Anspruch 4, worin die Pastenkomponente auch ein Konservierungsmittel, ein Färbemittel und einen Geschmacksstoff umfaßt.

6. Zusammensetzung nach Anspruch 1, worin die Gelkomponente 3,0 bis 6,5% Wasserstoffperoxid; 1 bis 3% des Copolymers; und 0,3 bis 1,5% des neutralisierenden Mittels umfaßt und worin die Pastenkomponente 20 bis 40% des Natriumbicarbonats; 2 bis 4% des Salzes; 15 bis 25% des Feuchthaltemittels; 1,0 bis 2,0% des Stabilisators; und 1,5 bis 20% des stabilisierenden Poliermittels umfaßt.

7. Zusammensetzung, die bei der Bekämpfung von Zahnfleischerkrankungen brauchbar ist, umfassend:
(a) eine nichtneutralisierte Gelkomponente, die im wesentlichen besteht aus 0,1 bis 10,0% Wasserstoffperoxid; 0,05 bis 5,0% eines mit Polyallylsucrose vernetzten Acrylsäure-Copolymers; 2 bis 80% eines Polyols, ausgewählt aus der Gruppe bestehend aus Glycerin, Sorbit (70%ige Lösung), Propylenglykol, Polypropylenglykol, Polyethylenglykol, einem ethoxylierten niederen Fettalkohol, einem propoxylierten niederen Fettalkohol und Gemischen davon; und gereinigtem Wasser;
(b) eine Pastenkomponente umfassend: (i) 2 bis 60% Natriumbicarbonat; (ii) 0 bis 6% eines Salzes, ausgewählt aus der Gruppe bestehend aus NaCl, KCl, MgCl₂, MgSO₄, Na₂SO₄ und K₂SO₄; (iii) 2 bis 60% eines Feuchthaltemittels, ausgewählt aus der Gruppe bestehend aus Glycerin, Sorbit, Polyethylenglykol, Propylenglykol, Polypropylenglykol, einem ethoxylierten niederen Fettalkohol, einem propoxylierten niederen Fettalkohol und Gemischen davon; (iv) 0,1 bis 5% eines Verdickungsmittelstabilisators, ausgewählt aus der Gruppe bestehend aus Cellulosegummi, Magnesiumaluminiumsilicat und Gemischen davon; (v) 1 bis 30% eines stabilisierenden Poliermittels, ausgewählt aus der Gruppe bestehend aus Bentonit, Titandioxid, Siliciumdioxid, Magnesiumoxid und Gemischen davon; und (vi) gereinigtes Wasser; wobei die Pastenkomponente mit der Gelkomponente unmittelbar vor der Verwendung vereinigt wird.

8. Zusammensetzung nach Anspruch 7, wobei die Pastenkomponente auch eine fluorhaltige Verbindung, ausgewählt aus der Gruppe bestehend aus NaF, KF, Natriummonofluorphosphat, Kaliummonofluorphosphat, Natriumfluorosilicat, Natriumfluorozirconat und Gemischen davon, in einer Menge umfaßt, die ausreichend ist, um 200 bis 3000 ppm Fluor zu ergeben.

9. Zusammensetzung nach Anspruch 8, wobei die Paste ferner 0,1 bis 2,5% eines Schaummittels, ausgewählt aus der Gruppe bestehend aus Natriumlaurylsulfat, Natriumlauroylsarcosinat, Natrium-Kokosnußmonoglyceridsulfonat, Natrium-N-methyl-N-palmitoyl-laurid, einem Polysorbat, einem Poloxamer und Gemischen davon, umfaßt.

10. Zusammensetzung nach Anspruch 9, wobei die Paste ferner 1 bis 30% eines zusätzlichen Reinigungsmittels, ausgewählt aus der Gruppe bestehend aus Calciumsulfat, Calciumphosphat, Aluminiumhydroxid, Calciumcarbonat, Magnesiumcarbonat, Magnesiumsilicat und Gemischen davon, umfaßt.

11. Zusammensetzung nach Anspruch 10, worin die Pastenkomponente auch ein Konservierungsmittel, ein Färbemittel und einen Geschmacksstoff umfaßt.

12. Zusammensetzung nach Anspruch 7, worin die Gelkomponente 3,0 bis 6,5% Wasserstoffperoxid; und 20 bis 60% des Polyols enthält und worin die Pastenkomponente 20 bis 40% des Natriumbicarbonats; 2 bis 4% des Salzes; 15 bis 25% des Feuchthaltemittels; 1,0 bis 2,0% des Stabilisators; und 1,5 bis 20% des stabilisierenden Poliermittels enthält.

13. Zusammensetzung, die bei der Bekämpfung von Zahnfleischerkrankungen brauchbar ist, umfassend:
(a) eine Gelkomponente umfassend: (i) 2 bis 25% Harnstoffperoxid; (ii) 0,0 bis 5,0% eines mit Polyallylsucrose vernetzten Acrylsäure-Copolymers; wobei der Rest Glycerin ist; und
(b) eine Pastenkomponente umfassend: (i) 2 bis 60% Natriumbicarbonat; (ii) 0 bis 6% eines Salzes, ausgewählt aus der Gruppe bestehend aus NaCl, KCl, MgCl₂, MgSO₄, Na₂SO₄ und K₂SO₄; (iii) 2 bis 60% eines Feuchthaltemittels, ausgewählt aus der Gruppe bestehend aus Glycerin, Sorbit, Polyethylenglykol, Polypropylenglykol, Propylenglykol, einem ethoxylierten niederen Fettalkohol, einem propoxylierten niederen Fettalkohol und Gemischen davon; (iv) 0,1 bis 5% eines Verdickungsmittelstabilisators, ausgewählt aus der Gruppe bestehend aus Cellulosegummi, Magnesiumaluminiumsilicat und Gemischen davon; (v) 1 bis 30% eines stabilisierenden Poliermittels, ausgewählt aus der Gruppe bestehend aus Bentonit, Titandioxid, Siliciumdioxid, Magnesiumoxid und Gemischen davon; und (vi) gereinigtes Wasser, wobei die Paste und die Gelkomponente unmittelbar vor der Verwendung vereinigt werden.

14. Zusammensetzung nach Anspruch 13, worin die Pastenkomponente auch eine fluorhaltige Verbindung, ausgewählt aus der Gruppe bestehend aus NaF, KF, Natriummonofluorphosphat, Kaliummonofluorphosphat, Natriumfluorosilicat, Natriumfluorozirconat und Gemischen davon, in einer Menge umfaßt, die ausreichend ist, um 200 bis 3000 ppm Fluor zu ergeben.

15. Zusammensetzung nach Anspruch 14, wobei die Paste ferner 0,1 bis 2,5% eines Schaummittels, ausgewählt aus der Gruppe bestehend aus Natriumlaurylsulfat, Natriumlauroylsarcosinat, Natrium-Kokosnußmonoglyceridsulfonat, Natrium-N-methyl-N-palmitoyl-laurid, einem Polysorbat, einem Poloxamer und Gemischen davon, umfaßt.

16. Zusammensetzung nach Anspruch 15, wobei die Paste ferner 1 bis 30% eines zusätzlichen Reinigungsmittels, ausgewählt aus der Gruppe bestehend aus Calciumsulfat, Calciumphosphat, Aluminiumhydroxid, Calciumcarbonat, Magnesiumcarbonat, Magnesiumsilicat und Gemischen davon, umfaßt.

17. Zusammensetzung nach Anspruch 16, worin die Pastenkomponente auch ein Konservierungsmittel, ein Färbemittel und einen Geschmacksstoff umfaßt.

18. Zusammensetzung nach Anspruch 13, worin die Gelkomponente 8 bis 12% Harnstoffperoxid; 1 bis 3% des Acrylsäure-Copolymers; und Glycerin enthält und worin die Pastenkomponente 20 bis 40% des Natriumbicarbonats; 2 bis 4% des Salzes; 15 bis 25% des Feuchthaltemittels; 1,0 bis 2,0% des Stabilisators; und 1,5 bis 20% des stabilisierenden Poliermittels umfaßt.

19. Zusammensetzung nach Anspruch 5, worin das Gelcopolymer ausgewählt ist aus der Gruppe bestehend aus Carboxyvinylpolymeren, der Gelstabilisator Hydroxypropylcellulose und das Neutralisationsmittel NaOH ist; das Salz NaCl ist, das Pastenfeuchthaltemittel Glycerin ist, das stabilisierende Poliermittel ausgewählt ist aus der Gruppe bestehend aus TiO₂, Siliciumdioxid, Bentonit und Gemischen davon; die fluorierte Verbindung NaF ist, das Schaummittel Natriumlaurylsulfat ist und das Konservierungsmittel ausgewählt ist aus der Gruppe bestehend aus Methyl- und Propylparaben.

20. Zusammensetzung nach Anspruch 11, worin das Gelcopolymer ein Carboxyvinylpolymer und das Polyol Glycerin ist; das Salz NaCl ist, das Pastenfeuchthaltemittel Glycerin ist, das stabilisierende Poliermittel ausgewählt ist aus der Gruppe bestehend aus TiO₂, Siliciumdioxid, Bentonit und Gemischen davon; die fluorierte Verbindung NaF ist, das Schaummittel Natriumlaurylsulfat ist, und das Konservierungsmittel ausgewählt ist aus der Gruppe bestehend aus Methyl- und Propylparaben.

21. Zusammensetzung nach Anspruch 17, worin das Polymer ein Carboxyvinylpolymer ist; das Salz NaCl ist, das Pastenfeuchthaltemittel Glycerin ist, das stabilisierende Poliermittel ausgewählt ist aus der Gruppe bestehend aus TiO₂, Siliciumdioxid, Bentonit und Gemischen davon; die fluorierte Verbindung NaF ist, das Schaummittel Natriumlaurylsulfat ist, und das Konservierungsmittel ausgewählt ist aus der Gruppe bestehend aus Methyl- und Propylparaben.

22. Kombination aus einem Zweikammerbehälter und einer Zusammensetzung, bestehend aus einer Gelkomponente und einer Pastenkomponente, welche in dem Behälter enthalten sind, wobei die Kombination zur Verwendung bei der Bekämpfung von Zahnfleischerkrankungen geeignet ist, wobei der Behälter umfaßt:
(a) eine erste Kammer, welche das Gel enthält, wobei das Gel umfaßt (i) 1 bis 10% Wasserstoffperoxid; (ii) 0,05 bis 5,0% eines wasserdispergierbaren Copolymers aus mit Polyallylsucrose vernetzter Acrylsäure; (iii) 0,0 bis 2,0% eines nichtionischen Cellulosestabilisators; (iv) eine Menge eines neutralisierenden Mittels, die ausreichend ist, um den pH-Wert des Gels auf 3,0 bis 6,0 einzustellen, wobei das neutralisierende Mittel ausgewählt ist aus der Gruppe bestehend aus NaOH, KOH, Triethanolamin, Diisopropylamin und Ammoniak; und (v) Wasser; so daß das Gel unmittelbar bei Berührung mit einer schwach alkalischen Umgebung, die einen starken Elektrolyten enthält, sich verflüssigt, wodurch die Freisetzung von bakterizid wirksamen Mengen von naszierendem Sauerstoff verursacht wird; wobei das Gel eine ausreichende Viskosität hat, um sich selbst auf den Borsten einer Zahnbürste zu tragen und ausreichende Fluidität hat, um aus dem Behälter abgegeben zu werden; wobei die erste Kammer eine Öffnung zur Abgabe von kontrollierten Mengen des Gels hat;
(b) eine zweite Kammer, welche die Paste enthält, wobei die Paste umfaßt (i) 2 bis 60 Gew.-% Natriumbicarbonat; (ii) 0 bis 6% eines Salzes, ausgewählt aus der Gruppe bestehend aus NaCl, KCl, MgCl₂, MgSO₄, Na₂SO₄, K₂SO₄ und Gemischen davon; (iii) 2 bis 60% eines Feuchthaltemittels, ausgewählt aus der Gruppe bestehend aus Glycerin, Sorbit, Polyethylenglykol, Polypropylenglykol, einem ethoxylierten niederen Fettalkohol, einem propoxylierten niederen Fettalkohol und Gemischen davon; (iv) 0,1 bis 5,0% eines Verdickungsmittelstabilisators, ausgewählt aus der Gruppe bestehend aus Cellulosegummi, Magnesiumaluminiumsilicat und Gemischen davon; (v) 1 bis 30% eines stabilisierenden Poliermittels, ausgewählt aus der Gruppe bestehend aus Bentonit, Titandioxid, Siliciumdioxid, Magnesiumoxid und Gemischen davon; und (vi) gereinigtes Wasser; wobei die Paste eine ausreichende Viskosität hat, um sich selbst auf den Borsten einer Zahnbürste zu tragen und eine ausreichende Fluidität hat, um aus dem Behälter abgegeben zu werden; wobei die zweite Kammer eine Öffnung zur Abgabe von kontrollierten Mengen der Paste im wesentlichen gleichzeitig mit der Abgabe des Gels hat; wobei die Öffnung der ersten Kammer und die Öffnung der zweiten Kammer so eingerichtet sind, daß sie das Gel bzw. die Paste am gleichen Verwendungsort abgeben, wobei die erste und die zweite Kammer einen gemeinsamen Wandbereich haben und die Öffnungen im wesentlichen aneinandergrenzen.

23. Kombination aus einem Zweikammerbehälter und einer Zusammensetzung, bestehend aus einer Gelkomponente und einer Pastenkomponente, wobei die Zusammensetzung in dem Behälter enthalten ist, wobei die Kombination zur Bekämpfung von Zahnfleischerkrankungen geeignet ist, wobei der Behälter umfaßt:
(a) eine erste Kammer, welche das Gel enthält, wobei das Gel ein nicht neutralisiertes Gel ist und 0,1 bis 10% Wasserstoffperoxid; 0,05 bis 5,0% eines mit Polyallylsucrose vernetzten Acrylsäure-Copolymers; 2 bis 80% eines Polyols, ausgewählt aus der Gruppe bestehend aus Glycerin, Sorbit in 0 bis 70%iger Lösung, Propylenglykol, Polypropylenglykol, Polyethylenglykol, einem ethoxylierten niederen Fettalkohol, einem propoxylierten niederen Fettalkohol und Gemischen davon; und gereinigtes Wasser umfaßt; so daß das Gel unmittelbar bei Berührung mit einer schwach alkalischen Umgebung, die einen starken Elektrolyten enthält, sich verflüssigt, wodurch die Freisetzung von bakterizid wirksamen Mengen von naszierendem Sauerstoff verursacht wird; wobei das Gel eine ausreichende Viskosität hat, um sich selbst auf den Borsten einer Zahnbürste zu tragen und ausreichende Fluidität hat, um aus dem Behälter abgegeben zu werden; wobei die erste Kammer eine Öffnung zur Abgabe von kontrollierten Mengen des Gels hat;
(b) eine zweite Kammer, welche die Paste enthält, wobei die Paste umfaßt (i) 2 bis 60 Gew.-% Natriumbicarbonat; (ii) 0 bis 6% eines Salzes, ausgewählt aus der Gruppe bestehend aus NaCl, KCl, MgCl₂, MgSO₄, Na₂SO₄, K₂SO₄ und Gemischen davon; (iii) 2 bis 60% eines Feuchthaltemittels, ausgewählt aus der Gruppe bestehend aus Glycerln, Sorbit, Polyethylenglykol, Polypropylenglykol, einem ethoxylierten niederen Fettalkohol, einem propoxylierten niederen Fettalkohol und Gemischen davon; (iv) 0,1 bis 5,0% eines Verdickungsmittelstabilisators, ausgewählt aus der Gruppe bestehend aus Cellulosegummi, Magnesiumaluminiumsilicat und Gemischen davon; (v) 1 bis 30% eines stabilisierenden Poliermittels, ausgewählt aus der Gruppe bestehend aus Bentonit, Titandioxid, Siliciumdioxid, Magnesiumoxid und Gemischen davon; und (vi) gereinigtes Wasser; wobei die Paste eine ausreichende Viskosität hat, um sich selbst auf den Borsten einer Zahnbürste zu tragen und eine ausreichende Fluidität hat, um aus dem Behälter abgegeben zu werden; wobei die zweite Kammer eine Öffnung zur Abgabe kontrollierter Mengen der Paste im wesentlichen gleichzeitig mit der Abgabe des Gels hat; wobei die Öffnung der ersten Kammer und die Öffnung der zweiten Kammer so eingerichtet sind, daß sie das Gel bzw. die Paste am gleichen Verwendungsort abgeben, wobei die erste und die zweite Kammer einen gemeinsamen Wandbereich haben und die Öffnungen im wesentlichen aneinandergrenzen.

24. Kombination aus einem Zweikammerbehälter und einer Zusammensetzung, die aus einer Gelkomponente und einer Pastenkomponente besteht, wobei die Zusammensetzung in dem Behälter enthalten ist, wobei die Kombination zur Bekämpfung von Zahnfleischerkrankungen geeignet ist, wobei der Behälter umfaßt:
(a) eine erste Kammer, welche das Gel enthält, wobei das Gel umfaßt 2 bis 25% Harnstoffperoxid; 0,0 bis 3,5% eines mit Polyallylsucrose vernetzten Acrylsäure-Copolymers; und Glycerin; so daß das Gel unmittelbar bei Berührung mit einer schwach alkalischen Umgebung, die einen starken Elektrolyten enthält, sich verflüssigt, wodurch die Freisetzung von bakterizid wirksamen Mengen von naszierendem Sauerstoff verursacht wird; wobei das Gel eine ausreichende Viskosität hat, um sich selbst auf den Borsten einer Zahnbürste zu tragen und eine ausreichende Fluidität hat, um aus dem Behälter abgegeben zu werden; wobei die erste Kammer eine Öffnung zur Abgabe kontrollierter Mengen des Gels hat;
(b) eine zweite Kammer, welche die Paste enthält, wobei die Paste umfaßt (i) 2 bis 60 Gew.-% Natriumbicarbonat; (ii) 0 bis 6% eines Salzes, ausgewählt aus der Gruppe bestehend aus NaCl, KCl, MgCl₂, MgSO₄, Na₂SO₄, K₂SO₄ und Gemischen davon; (iii) 2 bis 60% eines Feuchthaltemittels, ausgewählt aus der Gruppe bestehend aus Glycerin, Sorbit, Polyethylenglykol, Polypropylenglykol, einem ethoxylierten niederen Fettalkohol, einem propoxylierten niederen Fettalkohol und Gemischen davon; (iv) 0,1 bis 5,0% eines Verdickungsmittelstabilisators, ausgewählt aus der Gruppe bestehend aus Cellulosegummi, Magnesiumaluminiumsilicat und Gemischen davon; (v) 1 bis 30% eines stabilisierenden Poliermittels, ausgewählt aus der Gruppe bestehend aus Bentonit, Titandioxid, Siliciumdioxid, Magnesiumoxid und Gemischen davon; und (vi) gereinigtes Wasser; wobei die Paste eine ausreichende Viskosität hat, um sich selbst auf den Borsten einer Zahnbürste zu tragen und eine ausreichende Fluidität hat, um aus dem Behälter abgegeben zu werden; wobei die zweite Kammer eine Öffnung zur Abgabe kontrollierter Mengen der Paste im wesentlichen gleichzeitig mit der Abgabe des Gels hat; wobei die Öffnung der ersten Kammer und die Öffnung der zweiten Kammer so eingerichtet sind, daß sie das Gel bzw. die Paste am gleichen Verwendungsort abgeben, wobei die ersten und zweiten Kammern einen gemeinsamen Wandbereich haben und die Öffnungen im wesentlichen aneinandergrenzen.

25. Kombination nach Anspruch 22, worin das Gel mehr als 1,2 und bis zu 5,0% des Copolymers enthält und der Zweikammerbehälter ausgewählt ist aus der Gruppe bestehend aus Zweikammerquetschtuben mit flexiblen Seitenwänden, Zweikammer-Druckbehältern, Zweikammerpumpen und Kombinationen von zwei Einzelkammertuben.

26. Kombination nach Anspruch 23, worin der Zweikammerbehälter ausgewählt ist aus der Gruppe bestehend aus Zweikammerquetschtuben mit flexiblen Seitenwänden, Zweikammer-Druckbehältern, Zweikammerpumpen und Kombinationen von zwei Einzelkammertuben.

27. Kombination nach Anspruch 24, worin der Zweikammerbehälter ausgewählt ist aus der Gruppe bestehend aus Zweikammerquetschtuben mit flexiblen Seitenwänden, Zweikammer-Druckbehältern, Zweikammerpumpen und Kombinationen von zwei Einzelkammertuben.

28. Zusammensetzung, die bei der Bekämpfung von Zahnfleischerkrankungen brauchbar ist, umfassend:
(a) eine Gelkomponente, umfassend eine wirksame Menge Peroxid, ausgewählt aus der Gruppe bestehend aus Wasserstoffperoxid und Harnstoffperoxid; ein wasserdispergierbares Polymer, ausgewählt aus der Gruppe bestehend aus einem Copolymer aus mit Polyallylsucrose vernetzter Acrylsäure, einem Polyuronsäurekolloid, einem Carboxymethylenkolloid, einem teilweise hydrolysierten Polyacrylat, einem teilweise hydrolysierten Polymethacrylat, einem teilweise hydrolysierten Acrylat-Methacrylat-Copolymer, und einem Polyoxyethylen-Polyoxypropylen-Blockcopolymer; einen nichtionischen Cellulosegummistabilisator; gereinigtes Wasser; und eine Menge eines neutralisierenden Mittels, ausgewählt aus der Gruppe bestehend aus Natriumhydroxid, Kaliumhydroxid, Triethanolamin, Diisopropylamin und Ammoniak, welche ausreichend ist, um den pH-Wert des Gels auf 3 bis 6 anzuheben; wobei das Gel eine ausreichende Viskosität hat, um sich selbst auf den Borsten einer Zahnbürste zu tragen und eine ausreichende Fluidität hat, um aus einer Tube mit quetschbarer Seitenwand beim Zusammendrücken abgegeben zu werden; und
(b) eine Pastenkomponente, umfassend wirksame Mengen von Natriumbicarbonat und einem Salz, ausgewählt aus der Gruppe bestehend aus Natriumchlorid und Magnesiumsulfat, mindestens einen Verdickungsmittelstabilisator, ausgewählt aus der Gruppe bestehend aus Cellulosegummi und Magnesiumaluminiumsilicat, ein körperverleihendes Mittel, gereinigtes Wasser, mindestens ein Reinigungs-Poliermittel, ausgewählt aus der Gruppe bestehend aus Calciumsulfat, Calciumphosphat und Aluminiumhydroxid, und ein Schaummittel, wobei die Paste eine ausreichende Viskosität hat, um sich selbst auf den Borsten einer Zahnbürste zu tragen und eine ausreichende Fluidität hat, um aus einer Tube mit quetschbarer Seitenwand durch Zusammendrücken abgegeben zu werden, wobei das Gel und die Pastenkomponenten unmittelbar vor der Verwendung vermischt werden.

29. Zusammensetzung nach Anspruch 28, worin die Gelkomponente umfaßt 1 bis 10 Gew.-% H₂O₂, 0,05 bis 1,2 Gew.-% Acrylsäure-Copolymer und 0,1 bis 1,5 Gew.-% nichtionischen Cellulosestabilisator, wobei der Cellulosestabilisator ausgewählt ist aus der Gruppe bestehend aus Hydroxyethylcellulose, Hydroxypropylcellulose und Hydroxypropylmethylcellulose, und worin die Pastenkomponente 10 bis 50 Gew.-% Natriumbicarbonat, 0 bis 6 Gew.-% Salz, 1 bis 3 Gew.-% von mindestens einem von Cellulosegummi und Magnesiumaluminiumsilicat, 5 bis 30 Gew.-% eines körperverleihenden Mittels, 1 bis 40 Gew.-% des Reinigungs-Poliermittels und 0,1 bis 2,5 Gew.-% Schaummittel umfaßt.

30. Verfahren zum Reinigen von Zähnen, umfassend das Auspressen einer Gelkomponente nach den Ansprüchen 1 und 7, die Wasserstoffperoxid als aktiven Bestandteil umfaßt, wobei die erste Komponente zur oralen Anwendung geeignet ist;
das Auspressen einer Pastenkomponente nach den Ansprüchen 1 und 7, die Natriumbicarbonat als wirksamen Bestandteil umfaßt, wobei die zweite Komponente zur oralen Anwendung geeignet ist;
das In-Berührung-bringen der ersten Komponente und der zweiten Komponente miteinander auf einer Zahnbürste, und das Bürsten der Zähne, wobei die erste und zweite Komponente gleichzeitig als Reinigungsmedium verwendet werden.

31. Verfahren nach Anspruch 30, worin die erste und zweite Komponente zusammen auf die Zahnbürste ausgepresst werden.

32. Verfahren nach Anspruch 30, worin die erste und zweite Komponente getrennt voneinander ausgepresst und auf die Bürste aufgetragen werden.

33. Verfahren nach Anspruch 30, worin das Bürsten unmittelbar nach dem Auspressen der Komponenten und ihrem Auftragen auf die Zahnbürste stattfindet.

34. Verfahren nach Anspruch 30, worin die erste Komponente 0,1 bis 10% H₂O₂ enthält.

35. Verfahren nach Anspruch 30, worin die erste Komponente ein nicht neutralisiertes Gel ist.

36. Verfahren nach Anspruch 30, worin die erste Komponente ein neutralisiertes Gel ist.

37. Verfahren nach Anspruch 30, worin die zweite Komponente 2 bis 60 Gew.-% Natriumbicarbonat enthält.

38. Verfahren nach Anspruch 30, worin die zweite Komponente eine Paste ist.

39. Verfahren nach Anspruch 30, worin die erste Komponente ein Gel ist, das als Geliermittel einen Bestandteil umfaßt, ausgewählt aus der Gruppe bestehend aus: (a) Copolymeren aus mit Polyallylsucrose vernetzter Acrylsäure; (b) einem Kolloid aus einer organischen polymeren Säure; und (c) einem Polyoxyethylen/Polyoxypropylen-Blockcopolymer.

40. Verfahren nach Anspruch 30, worin die zweite Komponente eine Paste ist, die ein Verdickungsmittel/Stabilisator umfaßt.

41. Verfahren nach Anspruch 40, worin die Pastenkomponente ferner ein körperverleihendes Mittel umfaßt.

42. Verfahren nach Anspruch 40, worin die Pastenkomponente ferner ein zusätzliches Zahnreinigungsmittel umfaßt.

43. Verfahren nach Anspruch 40, worin die Pastenkomponente ferner eine fluorhaltige Verbindung mit Antikarieswirkung umfaßt.

44. Verfahren nach Anspruch 35, worin die Gelkomponente ferner ein Copolymer aus mit Polyallylsucrose vernetzter Acrylsäure als Geliermittel umfaßt.

45. Verfahren nach Anspruch 36, worin die Gelkomponente ferner ein Copolymer aus mit Polyallylsucrose vernetzter Acrylsäure als Geliermittel und ein Polyol, ausgewählt aus der Gruppe bestehend aus Glycerin, Sorbit, Propylenglykol, Polypropylenglykol, Polyethylenglykol, einem ethoxylierten niederen Fettalkohol und einem propoxylierten niederen Fettsäurealkohol umfaßt.

## Revendications

1. Composition utile pour combattre des maladies des gencives, comprenant:
(a) un composant à consistance de gel comprenant (i) de 0,1 à 10%
en poids de peroxyde d'hydrogène; (ii) de 0,05 à 5,0 % en poids d'un copolymère dispersible dans l'eau d'acide acrylique réticulé avec du saccharose de polyallyle ; (iii) de 0,0 à 2,0 % en poids d'un stabilisateur non-ionique en cellulose; (iv) un agent neutralisant choisi dans le groupe consistant en hydroxyde de sodium, hydroxyde de potassium, triéthanolamine, diisopropylamine et ammoniac selon une quantité suffisante pour faire monter le pH du gel à environ 3 à 6; et (v) de l'eau purifiée;
(b) un composant à consistance de pâte comprenant : (i) de 2 à 60% de
bicarbonate de sodium; (ii) de 0 à 6% d'un sel choisi dans le groupe consistant en NaCl, KCl, MgCl₂, MgSO₄, Na₂SO₄ et K₂SO₄; (iii) de 2 à 60% d'un humectant choisi dans le groupe consistant en glycérine, sorbitol, polyéthylène glycol, propylène glycol, polypropylène glycol, un alcool gras inférieur éthoxylaté, un alcool gras inférieur propoxylaté et des mélanges de ceux-ci; (iv) de 0,1 à 5% d'un épaississeur stabilisateur choisi dans le groupe consistant en gomme de cellulose, silicate de magnésium aluminium et leurs mélanges; (v) de 1 à 30% d'un agent stabilisateur et de polissage choisi dans le groupe consistant en bentonite, dioxyde de titane, silice, oxyde de magnésium et leurs mélanges; et (vi) de l'eau purifiée; ledit composant à consistance de pâte et ledit composant à consistance de gel étant combinés immédiatement avant l'utilisation.

2. Composition selon la revendication 1, ledit composant à consistance de pâte comprenant également un composé contenant du fluor choisi dans le groupe consistant en NaF, KF, monofluorophosphate de sodium, monofluorophosphate de potassium, fluorosilicate de sodium, fluorozirconate de sodium et leurs mélanges selon une quantité suffisante pour fournir de 200 à 3000 ppm de fluor; ladite composition étant également efficace contre les caries.

3. Composition selon la revendication 2, ladite pâte comprenant en outre de 0,1 à 2,5% d'un agent moussant choisi dans le groupe consistant en sulfate de lauryle sodium, sarcosinate de lauroyle sodium, sulfonate de monoglycéride de copra sodium, lauride de N-méthyle-N-palmitoyle sodium, un polysorbate, un poloxamère et leurs mélanges.

4. Composition selon la revendication 3, ladite pâte comprenant en outre de 1 à 30% d'un agent de nettoyage additionnel choisi dans le groupe consistant en sulfate de calcium, phosphate de calcium, oxyde d'aluminium hydraté, carbonate de calcium, carbonate de magnésium, silicate de magnésium et leurs mélanges.

5. Composition selon la revendication 4, dans laquelle ledit composant à consistance de pâte comprend également un préservatif, un agent colorant et agent d'aromatisation.

6. Composition selon la revendication 1, dans laquelle ledit composant à consistance de gel comprend de 3,0 à 6,5% de peroxyde d'hydrogène; de 1 à 3% dudit copolymère; et de 0,3 à 1,5% dudit agent neutralisant et dans laquelle ledit composant à consistance de pâte comprend de 20 à 40% dudit bicarbonate de sodium; de 2 à 4% dudit sel; de 15 à 25% dudit humectant; de 1,0 à 2,0% dudit stabilisateur; et de 1,5 à 20% dudit agent stabilisateur et de polissage.

7. Composition utile pour combattre des maladies des gencives, comprenant:
(a) un composant à consistance de gel non neutralisé consistant
essentiellement en 0,1 à 10,0% de peroxyde d'hydrogène; 0,05 à 5,0% d'un copolymère d'acide acrylique réticulé avec du saccharose de polyallyle; 2 à 80% d'un polyol choisi dans le groupe consistant en glycérine, sorbitol (solution à 70%), propylène glycol, polypropylène glycol, polyéthylène glycol, un alcool gras inférieur éthoxylaté, un alcool gras inférieur propoxylaté et leurs mélanges; et de l'eau purifiée;
(b) un composant à consistance de pâte comprenant: (i) de 2 à 60% de
bicarbonate de sodium; (ii) de 0 à 6% d'un sel choisi dans le groupe consistant en NaCl, KCl, MgCl₂, MgSO₄, Na₂SO₄ et K₂SO₄; (iii) de 2 à 60% d'un humectant choisi dans le groupe consistant en glycérine, sorbitol polyéthylène glycol, propylène glycol, polypropylène glycol, un alcool gras inférieur éthoxylaté, un alcool gras inférieur propoxylaté et des mélanges de ceux-ci; (iv) de 0,1 à 5% d'un épaississeur stabilisateur choisi dans le groupe consistant en gomme de cellulose, silicate de magnésium aluminium et leurs mélanges; (v) de 1 à 30% d'un agent stabilisateur et de polissage choisi dans le groupe consistant en bentonite, dioxyde de titane, silice, oxyde de magnésium et leurs mélanges; et (vi ) de l'eau purifiée; ledit composant à consistance de pâte étant combiné avec ledit composant à consistance de gel immédiatement avant l'utilisation.

8. Composition selon la revendication 7, ledit composant à consistance de pâte comprenant également un composé contenant du fluor choisi dans le groupe consistant en NaF, KF, monofluorophosphate de sodium, monofluorophosphate de potassium, fluorosilicate de sodium, fluorozirconate de sodium et leurs mélanges, selon une quantité suffisante pour fournir de 200 à 3000 ppm de fluor.

9. Composition selon la revendication 8, ladite pâte comprenant en outre de 0,1 à 2,5% d'un agent moussant choisi dans le groupe consistant en sulfate de lauryle sodium, sarcosinate de lauroyle sodium, sulfonate de monoglycéride de copra sodium, lauride de N-méthyle-N-palmitoyle sodium, un polysorbate, un poloxamère et leurs mélanges.

10. Composition selon la revendication 9, ladite pâte comprenant en outre de 1 à 30% d'un agent de nettoyage additionnel choisi dans le groupe consistant en sulfate de calcium, phosphate de calcium, oxyde d'aluminium hydraté, carbonate de calcium, carbonate de magnésium, silicate de magnésium et leurs mélanges.

11. Composition selon la revendication 10, dans laquelle ledit composant à consistance de pâte comprend également un préservatif, un agent colorant et agent d'aromatisation.

12. Composition selon la revendication 7, dans laquelle ledit composant à consistance de gel contient de 3,0 à 6,5% de peroxyde d'hydrogène, et de 20 à 60% dudit polyol, et dans laquelle ledit composant à consistance de pâte comprend de 20 à 40% dudit bicarbonate de sodium; de 2 à 4% dudit sel; de 15 à 25% dudit humectant; de 1,0 à 2,0% dudit stabilisateur; et de 1,5 à 20% dudit agent stabilisateur et de polissage.

13. Composition utile pour combattre des maladies des gencives, comprenant:
(a) un composant à consistance de gel comprenant (i) de 2 à 25% de
peroxyde d'urée; (ii) de 0,0 à 5,0% d'un copolymère d'acide acrylique réticulé avec du saccharose polyallyle; le complément étant de la glycérine; et
(b) un composant à consistance de pâte comprenant: (i) de 2 à 60% de
bicarbonate de sodium; (ii) de 0 à 6% d'un sel choisi dans le groupe consistant en NaCl, KCl, MgCl₂, MgSO₄, Na₂SO₄ et K₂SO₄; (iii) de 2 à 60% d'un humectant choisi dans le groupe consistant en glycérine, sorbitol, polyéthylène glycol , polypropylène glycol, propylène glycol, un alcool gras inférieur éthoxylaté, un alcool gras inférieur propoxylaté et des mélanges de ceux-ci; (iv) de 0,1 à 5% d'un épaississeur stabilisateur choisi dans le groupe consistant en gomme de cellulose, silicate de magnésium aluminium et leurs mélanges; (v) de 1 à 30% d'un agent stabilisateur et de polissage choisi dans le groupe consistant en bentonite, dioxyde de titane, silice, oxyde de magnésium et leurs mélanges; et (vi) de l'eau purifiée, ledit composant à consistance de pâte et ledit composant à consistance de gel étant combinés immédiatement avant l'utilisation.

14. Composition selon la revendication 13, ledit composant à consistance de pâte comprenant également un composé contenant du fluor choisi dans le groupe consistant en NaF, KF, monofluorophosphate de sodium, monofluorophosphate de potassium, fluorosilicate de sodium, fluorozirconate de sodium et leurs mélanges, selon une quantité suffisante pour fournir de 200 à 3000 ppm de fluor.

15. Composition selon la revendication 14, ladite pâte comprenant en outre de 0,1 à 2,5% d'un agent moussant choisi dans le groupe consistant en sulfate de lauryle sodium, sarcosinate de lauroyle sodium, sulfonate de monoglycéride de copra sodium, lauride de N-méthyle-N-palmitoyle sodium, un polysorbate, un poloxamère et leurs mélanges.

16. Composition selon la revendication 15, ladite pâte comprenant en outre de 1 à 30% d'un agent de nettoyage additionnel choisi dans le groupe consistant en sulfate de calcium, phosphate de calcium, oxyde d'aluminium hydraté, carbonate de calcium, carbonate de magnésium, silicate de magnésium et leurs mélanges.

17. Composition selon la revendication 16, dans laquelle ledit composant à consistance de pâte comprend également un préservatif, un agent colorant et un agent d'aromatisation.

18. Composition selon la revendication 13, dans laquelle ledit composant à consistance de gel contient de 8 à 12% de peroxyde d'urée; de 1 à 3% dudit copolymère d'acide acrylique; et de la glycérine et dans laquelle ledit composant à consistance de pâte comprend de 20 à 40% dudit bicarbonate de sodium; de 2 à 4% dudit sel; de 15 à 25% dudit humectant; de 1,0 à 2,0% dudit stabilisateur; et de 1,5 à 20% dudit agent stabilisateur et de polissage.

19. Composition selon la revendication 5, dans laquelle ledit copolymère de gel est choisi dans le groupe consistant en polymères de carboxyvinyle, ledit stabilisateur de gel est de la hydroxypropyl-cellulose, et ledit neutralisant est NaOH; ledit sel est NaCl, ledit humectant de pâte de la glycérine, ledit agent stabilisateur et de polissage est choisi dans le groupe consistant en TiO₂, silice, bentonite et leurs mélanges; ledit composé fluoré est NaF, ledit agent moussant est du sulfate de lauryle sodium et ledit préservatif est choisi dans le groupe consistant en para-hydroxybenzoate de méthyle et de propyle.

20. Composition selon la revendication 11, dans laquelle ledit copolymère à consistance de gel est un polymère de carboxyvinyle et le polyol de la glycérine; ledit sel est NaCl, ledit humectant de pâte est de la glycérine, ledit agent stabilisateur et de polissage est choisi dans le groupe consistant en TiO₂, silice, bentonite et leurs mélanges; ledit composé fluoré est NaF, ledit agent moussant est du sulfate de lauryle sodium, et ledit préservatif est choisi dans le groupe consistant en para-hydroxybenzoate de méthyle et propyle.

21. Composition selon la revendication 17, dans laquelle ledit polymère est un polymère de carboxyvinyle, ledit sel est NaCl, ledit humectant de pâte est de la glycérine, ledit agent stabilisateur et de polissage est choisi dans le groupe consistant en TiO₂, silice, bentonite et leurs mélanges; ledit composé fluoré est NaF, ledit agent moussant est du sulfate de lauryle sodium, et ledit préservatif est choisi dans le groupe consistant en para-hydroxybenzoate de méthyle et propyle.

22. Combinaison d'un récipient à deux compartiments et d'une composition consistant en un composant à consistance de gel et un composant à consistance de pâte et contenus dans ledit récipient, ladite combinaison étant apte à être utilisée pour combattre les maladies des gencives; ledit récipient comprenant:
(a) un premier compartiment contenant ledit gel, ledit gel
comprenant (i) de 1 à 10% de peroxyde d'hydrogène; (ii) de 0,05 à 5,0 % de copolymère dispersible dans l'eau d'acide acrylique réticulé avec du saccharose de polyallyle; (iii) de 0,0 à 2,0 % d'un stabilisateur en cellulose non-ionique; (iv) une quantité d'un agent neutralisant suffisante pour ajuster le pH du gel de 3,0 à 6,0, ledit agent neutralisant étant choisi dans le groupe consistant en NaOH, KOH, triéthanolamine, diisopropylamine et ammoniac; et (v) de l'eau; de manière que le gel se liquéfie immédiatement après contact avec un environnement légèrement alcalin contenant un électrolyte fort, provoquant ainsi le dégagement de quantités efficaces sur le plan bactéricide d'oxygène naissant; ledit gel présentant une viscosité suffisante pour se supporter de lui-même sur les soies d'une brosse à dents et une fluidité suffisante pour être distribué à partir dudit récipient; ledit premier compartiment comportant un orifice pour distribuer des quantités contrôlées dudit gel;
(b) un second compartiment contenant ladite pâte, ladite pâte
comprenant: (i) de 2 à 60% en poids de bicarbonate de sodium; (ii) de 0 à 6% d'un sel choisi dans le groupe consistant en NaCl, KCl, MgCl₂, MgSO₄, Na₂SO₄ et K₂SO₄ et leurs mélanges; (iii) de 2 à 60% d'un humectant choisi dans le groupe consistant en glycérine, sorbitol, polyéthylène glycol, polypropylène glycol, un alcool gras inférieur éthoxylaté, un alcool gras inférieur propoxylaté et des mélanges de ceux-ci; (iv) de 0,1 à 5,0% d'un épaississeur-stabilisateur choisi dans le groupe consistant en gomme de cellulose, silicate de magnésium aluminium et leurs mélanges; (v) de 1 à 30% d'un agent stabilisateur et de polissage choisi dans le groupe consistant en bentonite, dioxyde de titane, silice, oxyde de magnésium et leurs mélanges; et (vi) de l'eau purifiée; ladite pâte présentant une viscosité suffisante pour se supporter d'elle-même sur les soies d'une brosse à dents et une fluidité suffisante pour être distribuée à partir dudit récipient; ledit second compartiment comportant un orifice pour distribuer des quantités contrôlées de ladite pâte sensiblement simultanément avec la distribution dudit gel; l'orifice dudit premier compartiment et l'orifice dudit second compartiment étant adaptés à distribuer ledit gel et ladite pâte respectivement au même point d'utilisation, lesdits premier et second compartiments comportant une portion de paroi commune et lesdits orifices étant sensiblement adjacents.

23. Combinaison d'un récipient à deux compartiments et d'une composition consistant en un composant à consistance de gel et un composant à consistance de pâte, ladite composition étant contenue dans ledit récipient, ladite combinaison étant apte à combattre les maladies des gencives; ledit récipient comprenant:
(a) un premier compartiment contenant ledit gel, ledit gel étant un
gel non neutralisé et comprenant de 0,1 à 10% de peroxyde d'hydrogène; de 0,05 à 5,0% d'un copolymère d'acide acrylique réticulé avec du saccharose de polyallyle; de 2 à 80% d'un polyol choisi dans le groupe consistant en glycérine, une solution de 0 à 70% de sorbitol, propylène glycol, polypropylène glycol, polyéthylène glycol, un alcool gras inférieur éthoxylaté, un alcool gras inférieur propoxylaté et leurs mélanges; et de l'eau purifiée; de manière que le gel se liquéfie immédiatement après contact avec un environnement légèrement alcalin contenant un électrolyte fort, provoquant ainsi le dégagement de quantités efficaces sur le plan bactéricide d'oxygène naissant; ledit gel présentant une viscosité suffisante pour se supporter de lui-même sur les soies d'une brosse à dents et une fluidité suffisante pour être distribué à partir dudit récipient; ledit premier compartiment comportant un orifice pour distribuer des quantités contrôlées dudit gel;
(b) un second compartiment contenant ladite pâte, ladite pâte
comprenant: (i) de 2 à 60% en poids de bicarbonate de sodium; (ii) de 0 à 6% d'un sel choisi dans le groupe consistant en NaCl, KCl, MgCl₂, MgSO₄, Na₃SO₄ et K₂SO₄ et leurs mélanges; (iii) de 2 à 60% d'un humectant choisi dans le groupe consistant en glycérine, sorbitol, polyéthylène glycol, polypropylène glycol, un alcool gras inférieur éthoxylaté, un alcool gras inférieur propoxylaté et des mélanges de ceux-ci; (iv) de 0,1 à 5,0% d'un épaississeur-stabilisateur choisi dans le groupe consistant en gomme de cellulose, silicate de magnésium aluminium et leurs mélanges; (v) de 1 à 30% d'un agent stabilisateur et de polissage choisi dans le groupe consistant en bentonite, dioxyde de titane, silice, oxyde de magnésium et leurs mélanges; et (vi) de l'eau purifiée; ladite pâte présentant une viscosité suffisante pour se supporter d'elle-même sur les soies d'une brosse à dents et une fluidité suffisante pour être distribuée à partir dudit récipient; ledit second compartiment comportant un orifice pour distribuer des quantités contrôlées de ladite pâte sensiblement simultanément avec la distribution dudit gel; l'orifice dudit premier compartiment et l'orifice dudit second compartiment étant adaptés à distribuer ledit gel et ladite pâte respectivement au même point d'utilisation, lesdits premier et second compartiments comportant une portion de paroi commune et lesdits orifices étant sensiblement adjacents.

24. Combinaison d'un récipient à deux compartiments et d'une composition consistant en un composant à consistance de gel et un composant à consistance de pâte, ladite composition étant contenue dans ledit récipient, ladite combinaison étant apte à combattre les maladies des gencives; ledit récipient comprenant:
(a) un premier compartiment contenant ledit gel, ledit gel
comprenant de 2 à 25% de peroxyde d'urée, de 0,0 à 3,5% d'un copolymère d'acide acrylique réticulé avec du saccharose de polyallyle; et de la glycérine; de manière que le gel se liquéfie immédiatement après contact avec un environnement légèrement alcalin contenant un électrolyte fort, provoquant ainsi le dégagement de quantités efficaces sur le plan bactéricide d'oxygène naissant; ledit gel présentant une viscosité suffisante pour se supporter de lui-même sur les soies d'une brosse à dents et une fluidité suffisante pour être distribué à partir dudit récipient; ledit premier compartiment comportant un orifice pour distribuer des quantités contrôlées dudit gel;
(b) un second compartiment contenant ladite pâte, ladite pâte
comprenant: (i) de 2 à 60% en poids de bicarbonate de sodium; (ii) de 0 à 6% d'un sel choisi dans le groupe consistant en NaCl, KCl, MgCl₂, MgSO₄, Na₂SO₄ et K₂SO₄ et leurs mélanges; (iii) de 2 à 60% d'un humectant choisi dans le groupe consistant en glycérine, sorbitol, polyéthylène glycol, polypropylène glycol, un alcool gras inférieur éthoxylaté, un alcool gras inférieur propoxylaté et des mélanges de ceux-ci; (iv) de 0,1 à 5,0% d'un épaississeur-stabilisateur choisi dans le groupe consistant en gomme de cellulose, silicate de magnésium aluminium et leurs mélanges; (v) de 1 à 30% d'un agent stabilisateur et de polissage choisi dans le groupe consistant en bentonite, dioxyde de titane, silice, oxyde de magnésium et leurs mélanges; et (vi) de l'eau purifiée; ladite pâte présentant une viscosité suffisante pour se supporter d'elle-même sur les soies d'une brosse à dents et une fluidité suffisante pour être distribuée à partir dudit récipient; ledit second compartiment comportant un orifice pour distribuer des quantités contrôlées de ladite pâte sensiblement simultanément avec la distribution dudit gel; l'orifice dudit premier compartiment et l'orifice dudit second compartiment étant adaptés à distribuer ledit gel et ladite pâte respectivement au même point d'utilisation, lesdits premier et second compartiments comportant une portion de paroi commune et lesdits orifices étant sensiblement adjacents.

25. Combinaison selon la revendication 22, dans laquelle ledit gel contient plus de 1,2 et jusqu'à 5,0% dudit copolymère et ledit récipient à deux compartiments est choisi dans le groupe consistant en des tubes pliables à deux compartiments à parois latérales flexibles, des récipients pressurisés à deux compartiments, des pompes à deux compartiments, et des combinaisons de deux tubes à compartiment unique.

26. Combinaison selon la revendication 23, dans lequel ledit récipient à deux compartiments est choisi dans le groupe consistant en des tubes pliables à deux compartiments à parois latérales flexibles, des récipients pressurisés à deux compartiments, des pompes à deux compartiments, et des combinaisons de deux tubes à compartiment unique.

27. Combinaison selon la revendication 24, dans laquelle ledit récipient à deux compartiments est choisi dans le groupe consistant en des tubes pliables à deux compartiments à parois latérales flexibles, des récipients pressurisés à deux compartiments, des pompes à deux compartiments, et des combinaisons de deux tubes à compartiment unique.

28. Composition utile pour combattre des maladies des gencives, comprenant:
(a) un composant à consistance de gel comprenant une quantité
efficace de peroxyde choisi dans le groupe consistant en peroxyde d'hydrogène et peroxyde d'urée; un polymère dispersible dans l'eau choisi dans le groupe consistant en un copolymère d'acide acrylique réticulé avec du saccharose de polyallyle, un colloïde d'acide polyuronique, un colloïde de carboxyméthylène, un polyacrylate partiellement hydrolysé, un polyméthacrylate partiellement hydrolysé, un copolymère d'acrylate méthacrylate partiellement hydrolysé, et un copolymère en masse de polyoxyéthylène-polyoxypropylène; un stabilisateur en gomme de cellulose non-ionique; de l'eau purifiée; et une quantité d'agent neutralisant choisi dans le groupe consistant en: hydroxyde de sodium, hydroxyde de potassium, triéthanolamine, diisopropylamine et ammoniac qui est suffisante pour faire monter le pH dudit gel entre 3 et 6; ledit gel présentant une viscosité suffisante pour se supporter de lui-même sur les soies d'une brosse à dents et une fluidité suffisante pour être distribué à partir d'un tube à parois latérales pliables quand on les serre; et
(b) un composant à consistance de pâte comprenant des quantités
efficaces de bicarbonate de sodium et d'un sel choisi dans le groupe consistant en chlorure de sodium et sulfate de magnésium, au moins un épaississeur-stabilisateur choisi dans le groupe consistant en gomme de cellulose et silicate de magnésium-aluminium, un agent donnant du corps, de l'eau purifiée, au moins un agent de nettoyage-polissage choisi dans le groupe consistant en sulfate de calcium, phosphate de calcium et oxyde d'aluminium hydraté, et un agent moussant, ladite pâte présentant une viscosité suffisante pour se supporter d'elle-même sur les soies d'une brosse à dents et une fluidité suffisante pour être distribuée à partir d'un tube à parois latérales pliables lorsqu'on les presse, lesdits composants à consistance de gel et à consistance de pâte étant mélangés immédiatement avant utilisation.

29. Composition selon la revendication 28, dans laquelle ledit composant à consistance de gel comprend de 1 à 10% de H₂O₂, de 0,05 à 1,2% de copolymère d'acide acrylique, et de 0,1 à 1,5% d'un stabilisateur à cellulose non-ionique, en poids, ledit stabilisateur en cellulose étant choisi dans le groupe consistant en hydroxyéthyle-cellulose, hydroxypropyle-cellulose et hydroxypropyle-méthylcellulose, et dans laquelle ledit composant à consistance de pâte comprend de 10 à 50% de bicarbonate de sodium, de 0 à 6% de sel, de 1 à 3% de l'un au moins parmi la gomme de cellulose et le silicate de magnésium-aluminium, de 5 à 30% d'un agent donnant du corps, de 1 à 40% dudit agent de nettoyage-polissage et de 0,1 à 2,5% d'agent moussant, en poids.

30. Procédé pour nettoyer les dents comprenant l'extrusion d'un composant à consistance de gel selon les revendications 1 et 7, comprenant du peroxyde d'hydrogène en tant qu'ingrédient actif, ledit premier composant étant apte à une utilisation orale;
l'extrusion d'un composant à consistance de pâte selon les revendications 1 et 7 comprenant du bicarbonate de sodium en tant qu'ingrédient actif, ledit second composant étant apte à une utilisation orale;
la mise en contact dudit premier composant et dudit second composant l'un avec l'autre sur une brosse à dents; et
le brossage desdites dents en utilisant lesdits premier et second composants concurremment en tant qu'agent de nettoyage.

31. Procédé selon la revendication 30, dans lequel lesdits premier et second composants sont extrudés ensemble sur la brosse à dents.

32. Procédé selon la revendication 30, dans lequel lesdits premier et second composants sont extrudés et placés séparément sur la brosse.

33. Procédé selon la revendication 30, dans lequel ledit brossage a lieu immédiatement l'extrusion desdits composants et leur mise en place sur ladite brosse à dents.

34. Procédé selon la revendication 30, dans lequel ledit premier composant contient de 0,1 à 10% de H₂O₂.

35. Procédé selon la revendication 30, dans lequel ledit premier composant est un gel non neutralisé.

36. Procédé selon la revendication 30, dans lequel ledit premier composant est un gel neutralisé.

37. Procédé selon la revendication 30, dans lequel ledit second composant contient de 2 à 60% en poids de bicarbonate de sodium.

38. Procédé selon la revendication 30, dans lequel ledit second composant est une pâte.

39. Procédé selon la revendication 30, dans lequel ledit premier composant est un gel comprenant en tant qu'agent de gélification une substance choisie dans le groupe consistant en: (a) des copolymères d'acide acrylique réticulé avec du saccharose de polyallyle; (b) un colloïde d'acide polymère organique; et (c) un copolymère de masse de polyoxyéthylène/polyoxypropylène.

40. Procédé selon la revendication 30, dans lequel ledit second composant est une pâte comprenant un épaississeur/stabilisateur.

41. Procédé selon la revendication 40, dans lequel ledit composant à consistance de pâte comprend en outre un agent donnant du corps.

42. Procédé selon la revendication 40, dans lequel ledit composant à consistance de pâte comprend en outre un agent additionnel de nettoyage des dents.

43. Procédé selon la revendication 40, dans lequel ledit composant à consistance de pâte comprend en outre un composé contenant du fluor présentant une activité anti-caries.

44. Procédé selon la revendication 35, dans lequel ledit composant à consistance de gel comprend en outre un copolymère d'acide acrylique réticulé avec du saccharose de polyallyle en tant qu'agent de gélification.

45. Procédé selon la revendication 36, dans lequel ledit composant à consistance de gel comprend en outre un copolymère d'acide acrylique réticulé avec du saccharose de polyallyle en tant qu'agent de gélification et un polyol choisi dans le groupe consistant en glycérine, sorbitol, propylène glycol, polypropylène glycol, polyéthylène glycol, un alcool gras inférieur éthoxylaté et un alcool acide gras inférieur propoxylaté.
